(19) Europäisches Patentamt
European Patent Office
Office européen des brevets

(11) Veröffentlichungsnummer: **0 192 176**
**A2**

(12) # EUROPÄISCHE PATENTANMELDUNG

(21) Anmeldenummer: **86101752.3**

(22) Anmeldetag: **12.02.86**

(51) Int. Cl.[4]: **C 07 D 501/46**
C 07 D 501/42, C 07 D 501/18
A 61 K 31/545, A 23 K 1/17

(30) Priorität: **22.02.85 DE 3506159**

(43) Veröffentlichungstag der Anmeldung:
**27.08.86 Patentblatt 86/35**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Boberg, Michael, Dr.**
**Untere Bergerheide 47**
**D-5600 Wuppertal 1(DE)**

(72) Erfinder: **Angerbauer, Rolf, Dr.**
**Sterntalerweg 29**
**D-5600 Wuppertal 1(DE)**

(72) Erfinder: **Metzger, Karl Georg, Dr.**
**Phalkestrasse 75**
**D-5600 Wuppertal 1(DE)**

(72) Erfinder: **Zeiler, Hans-Joachim, Dr.**
**Elsbeeker Strasse 46**
**D-5620 Velbert 15(DE)**

(72) Erfinder: **Schröck, Wilfried, Dr.**
**Pahlkestrasse 33**
**D-5600 Wuppertal 1(DE)**

(54) **Neue Cephalosporine, Verfahren zu ihrer Herstellung und ihre Verwendung in Arzneimitteln.**

(57) Die Erfindung betrifft neue Cephalosporine der Formel (I)

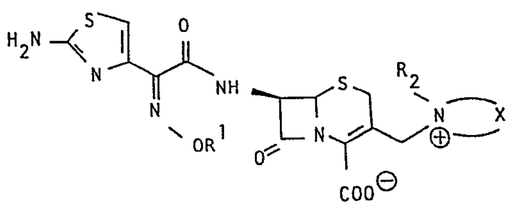

(I)

in der $R^1$ und $R^2$ die in der Beschreibung angegebene Bedeutung haben, Verfahren zu ihrer Herstellung sowie ihre Verwendung als Arzneimittel, insbesondere in der antibakteriellen Therapie.

EP 0 192 176 A2

Croydon Printing Company Ltd.

0192176

BAYER AKTIENGESELLSCHAFT 5090 Leverkusen, Bayerwerk
Konzernverwaltung RP
Patentabteilung Ad/Ke-c

# Neue Cephalosporine, Verfahren zu ihrer Herstellung und ihre Verwendung in Arzneimitteln

Die Erfindung betrifft neue Cephalosporine, Verfahren zu ihrer Herstellung sowie ihre Verwendung als Arzneimittel, insbesondere in der antibakteriellen Therapie.

Cephalosporine, die als Acylseitenkette einen 2-(2-Aminothiazol-4-yl)-2-alkoxyiminoessigsäurerest und in 3-Stellung einen Pyridiniummethyl-Rest tragen, sind aus EP 64 740 bekannt.

Die vorliegende Erfindung betrifft neue Cephalosporine der allgemeinen Formel (I)

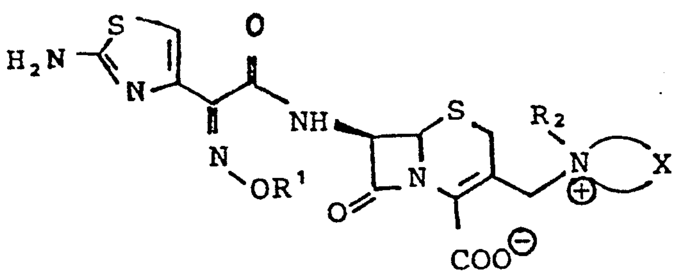

(I)

in welcher

$R^1$ - für geradkettiges oder verzweigtes, gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 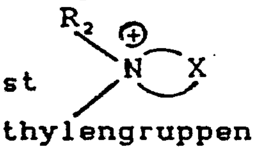 für die gegebenenfalls an den Ringmethylengruppen substituierten Gruppen

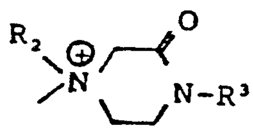

oder

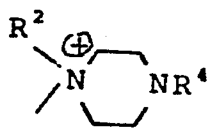 steht,

$R^3$ - für geradkettiges, verzweigtes oder cyclisches gesättigtes oder ungesättigtes Alkyl mit bis zu 10 C-Atomen steht, das gegebenenfalls substituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,
- durch Halogen, Cyano, $SO_3H$,

- durch die Gruppen 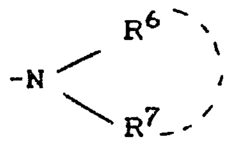 ,

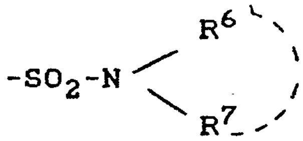 oder 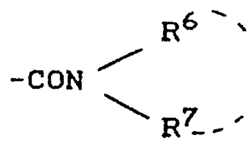 ,

wobei
$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der gegebenenfalls durch $C_1$-$C_4$-Alkyl substituiert ist,

oder

- durch die Gruppe $-SO_2-Y$ oder -O-Y, wobei
  Y - für $C_1$-$C_8$-Alkyl, Aryl mit 6 bis 10 C-Atomen
  oder
  - für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist
- durch OH, CHO, $OCONH_2$, $-NHCONH_2$,

- durch die Gruppen $COOR^5$, $NHCOR^5$, $COR^5$,
  wobei
  $R^5$ - für Wasserstoff, Alkyl mit bis zu 8 C-Atomen, Aryl mit 6 bis 10 C-Atomen oder $C_7$-$C_{14}$-Aralkyl steht,

- durch Halogen, Cyano, $SO_3H$,
- durch die Gruppen 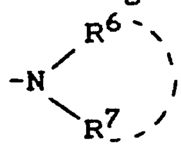 ,

- 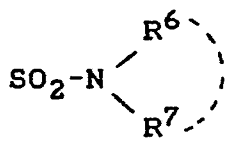 oder 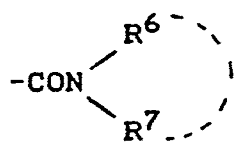

wobei
$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,
oder
- durch die Gruppe $-SO_2-Y$ oder $-O-Y$
wobei
Y - für $C_1$-$C_8$-Alkyl, Aryl mit 6 bis 10 C-Atomen oder
  - für $C_7$-$C_{14}$-Aralkyl steht,

b) für CHO, $SO_3H$ steht,

c) für $COOR^5$ steht, wobei $R^5$ die oben angegebene Bedeutung hat,

oder

d) für die Gruppe $-Z-R^8$ steht,

wobei

Z - für 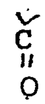 oder $-SO_2-$ steht und

$R^8$ - für geradkettiges, verzweigtes oder cyclisches gesättigtes oder ungesättigtes Alkyl mit bis zu 8 C-Atomen steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

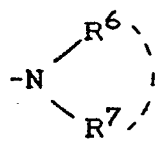 , 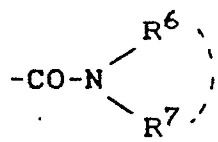 oder 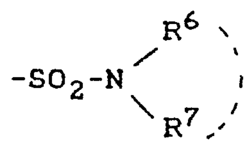

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder
- für die Gruppe 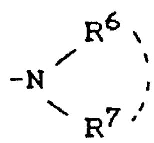 steht, wobei

$R^6$, $R^7$ die obengenannte Bedeutung haben.

Wenn die Reste 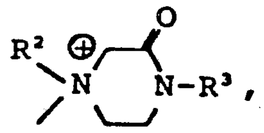, 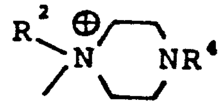 an den Ringmethylengruppen substituiert sind, dann durch bis zu vier, bevorzugt bis zu zwei gleiche oder unterschiedliche Substituenten wie:

a) geradkettiges, verzweigtes oder cyclisches gesättigtes oder ungesättigtes Alkyl mit bis zu 10 C-Atomen, das gegebenenfalls wieder substituiert ist

- durch -OH, CHO, $-OCONH_2$, $-NHCONH_2$
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen oder $C_7$-$C_{14}$-Aralkyl steht,

- durch Halogen, Cyano, $SO_3H$
- durch die Gruppen 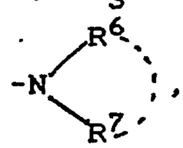,

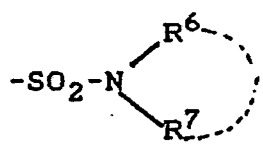 oder 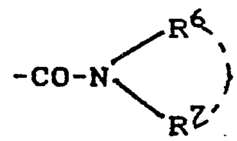

wobei

$R^6$ und $R^7$ unabhängig voneinander Wasserstoff oder gemeinsam oder einzeln für

$C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der gegebenenfalls durch $C_1$-$C_4$-Alkyl substituiert ist, oder

- durch die Gruppe $-SO_2$-Y oder -O-Y
  wobei
  Y - für $C_1$-$C_6$-Alkyl, Aryl mit 6 bis 10 C-Atomen oder
  - für $C_7$-$C_{14}$-Aralkyl steht

oder

b) die Gruppe -Z-$R^8$,
wobei

\ /
Z - für C oder $-SO_2-$ steht und
||
O

$R^8$ - für geradkettiges, verzweigtes oder cyclisches gesättigtes oder ungesättigtes Alkyl mit bis zu 6 C-Atomen steht, das gegebenenfalls durch Halogen, Hydroxy, Cyano, Alkoxy, Alkylthio mit jeweils 6 C-Atomen, Aryloxy, Arylthio (6-10-C-Atome), Carboxy, $C_1$-$C_6$-Alkoxycarbonyl, $-OCONH_2$-, $-NHCONH_2$, $-SO_3H$,

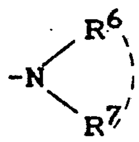 , 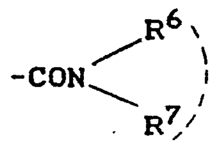 oder

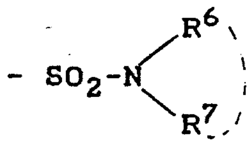

substituiert ist, wobei $R^6$, $R^7$ die oben angegebene Bedeutung haben

- für Aryl mit 6 bis 10 C-Atomen
- für $C_7$-$C_{14}$-Aralkyl
- für Heterocyclyl oder
- für die Gruppe 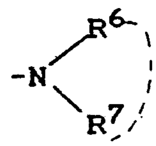 steht,

wobei $R^6$, $R^7$ wiederum die obengenannte Bedeutung haben.

c) -OH, CHO, $OCONH_2$, $NHCONH_2$,

d) die Gruppen $COOR^5$, $NHCOR^5$, $COR^5$,

wobei
$R^5$ - für Wasserstoff, Alkyl mit bis zu 8 C-Atomen, Aryl mit 6 bis 10 C-Atomen, oder $C_7$-$C_{14}$-Aralkyl steht,

e) Halogen, Cyano, $SO_3H$

f) die Gruppe 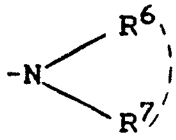 wobei

$R^6$ und $R^7$ die oben angegebene Bedeutung hat,

g) die Gruppe -O-Y, wobei

Y - für $C_1$-$C_6$-Alkyl, Aryl mit 6 bis 10 C-Atomen oder
- für $C_7$-$C_{14}$-Aralkyl steht.

Bevorzugte Verbindungen der allgemeinen Formel I sind solche, in welchen

$R^1$ -für geradkettiges, verzweigtes, gesättigtes oder ungesättigtes, gegebenenfalls durch Carboxy substituiertes Alkyl mit bis zu 4 C-Atomen steht,

$R^2$ -für geradkettiges oder verzweigtes Alkyl mit bis zu 4 C-Atomen steht, das gegebenenfalls substituiert ist durch Hydroxy, Carboxy, Methoxy, Methoxycarbonyl, Fluor, Chlor oder Brom,

der Rest 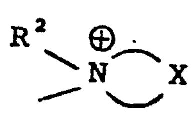 für die gegebenenfalls substituierten

Reste 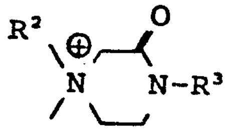 oder 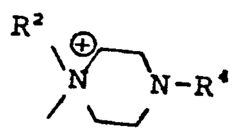 steht,

in denen

$R^3$- für geradkettiges, verzweigtes oder cyclisches, gesättigtes oder ungesättigtes Alkyl mit bis zu 8 C-Atomen steht, das gegebenenfalls substituiert ist

- durch OH, CHO, $OCONH_2$, $NHCONH_2$
- durch die Gruppen $COOR^5$, $NHCOR^5$, $COR^5$,

  wobei

  $R^5$ - für Wasserstoff, Alkyl mit bis zu 6-C-Atomen, Phenyl oder Benzyl steht,
- durch Fluor, Chlor, Brom oder $SO_3H$,
- durch die Gruppen - 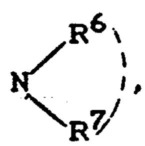, 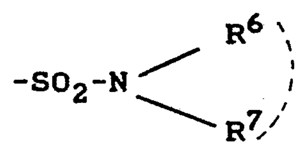

  oder 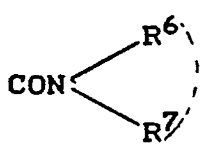 ,

  wobei

  $R^6$ und $R^7$ unabhängig voneinander für Wasserstoff, für $C_1$-$C_3$-Alkyl oder Phenyl stehen oder wobei die Substituenten $R^6$, $R^7$ gegebenenfalls mit dem Stickstoffatom einen 5- oder 6-gliedrigen Ring bilden, der als weitere Heteroatome ein Sauerstoff-, Schwefel- und/oder 1-2 Stickstoffatome enthalten kann und der gegebenenfalls durch $C_1$-$C_2$-Alkyl substituiert ist,

oder

- durch die Gruppe $-SO_2-Y$ oder -O-Y,

  wobei

  Y - für $C_1$-$C_6$-Alkyl, Phenyl oder Benzyl steht,

und

$R^4$ a) für geradkettiges, verzweigtes oder cyclisches, gesättigtes oder ungesättigtes Alkyl mit bis zu 8 C-Atomen steht, das mindestens einmal substituiert ist

- durch OH, CHO, $OCONH_2$, $NHCONH_2$
- durch die Gruppen $COOR^5$, $NHCOR^5$, $COR^5$
  wobei
  $R^5$ - für Wasserstoff, Alkyl mit bis zu 6 C-Atomen, Phenyl oder Benzyl steht,
- durch Fluor, Chlor, Brom oder $SO_3H$
- durch die Gruppen 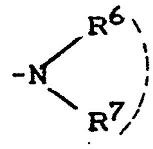 , 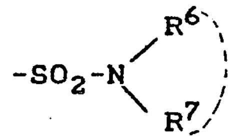 ,

  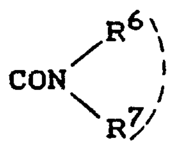

  wobei $R^6$ und $R^7$ die oben angegebene Bedeutung haben
- durch die Gruppe $-SO_2-Y$ oder -O-Y
  wobei
  Y - für $C_1$-$C_6$-Alkyl, Phenyl oder Benzyl steht, oder

b) für CHO oder $SO_3H$ steht,

c) für $COOR^5$ steht,
wobei
$R^5$ die oben angegebene Bedeutung hat,
oder

d) für die Gruppe $-Z-R^8$ steht,
wobei
Z - für 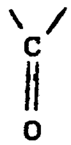 oder $-SO_2-$ steht und

$R^8$ - für geradkettiges, verzweigtes oder cyclisches, gesättigtes oder ungesättigtes Alkyl mit bis zu 6 C-Atomen, steht, das gegebenenfalls durch Fluor, Chlor, Brom, Hydroxy, Carboxy, Alkoxy oder, Alkylthio mit bis zu 3 C-Atomen, Phenyloxy, Phenylthio, Alkoxycarbonyl mit bis zu 3 C-Atomen, durch

$OCONH_2$, 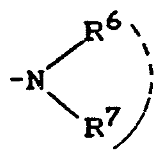 , 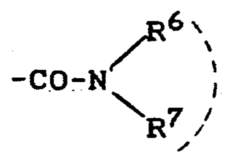

oder 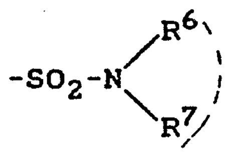 , substituiert ist,

wobei $R^6$ und $R^7$ die oben angegebene Bedeutung haben,

- für Phenyl,
- für Benzyl,
- für Pyridyl, Furyl, Thienyl, Thiazolyl, Pyrimidyl, Pyrazinyl, Pyridazinyl, Isoxazolyl, Oxazolyl, Morpholinyl, Piperidinyl, Imidazolyl, Pyrazolyl, Thiazolyl oder Piperazinyl steht,

oder

- für die Gruppe 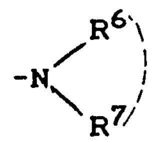 steht, wobei

$R^6$ und $R^7$ die oben angegebene Bedeutung haben.

Besonders bevorzugte Verbindungen der Formel I sind solche, in welchen

$R^1$ - für Methyl, Ethyl, Allyl oder 1-Carboxy-1-methylethyl steht,

$R^2$ - für Methyl, Ethyl, 2-Hydroxyethyl oder Carboxymethyl steht,

der Rest 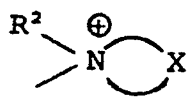 für die gegebenenfalls substituierten Gruppen

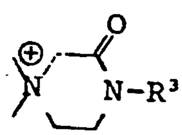 oder 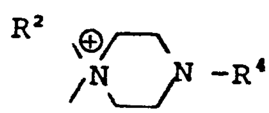

steht, worin

$R^3$ - für geradkettiges, verzweigtes oder cyclisches, gesättigtes oder ungesättigtes Alkyl mit bis zu 6 C-Atomen steht, das gegebenenfalls substituiert ist

- durch OH, CHO, $OCONH_2$, $NHCONH_2$
- durch die Gruppen $COOR^5$, $NHCOR^5$, oder $COR^5$, wobei

$R^5$ - für Wasserstoff, Alkyl mit bis zu 4 C-Atomen, Phenyl oder Benzyl steht,

- durch Fluor, Chlor oder $SO_3H$,
- durch die Gruppen 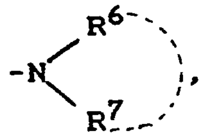, 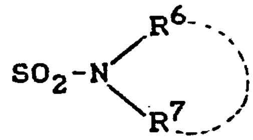

oder 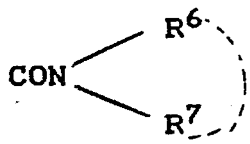,

wobei $R^6$, $R^7$ unabhänging voneinander für Wasserstoff, Methyl, Ethyl, Phenyl stehen oder mit dem Stickstoffatom einen Ring bilden wie Pyrrolidin, Piperazin, Piperidin, Morpholin oder Thiomorpholin, oder

- durch die Gruppe $-SO_2-Y$ oder $-O-Y$, wobei Y - für Alkyl (bis $C_4$), Phenyl oder Benzyl steht,

und

$R^4$ a) für geradkettiges, verzweigtes oder cyclisches gesättigtes oder ungesättigtes Alkyl mit bis zu 6 C-Atomen steht, das mindestens einmal substituiert ist

- durch OH, CHO, $OCONH_2$, $NHCONH_2$
- durch die Gruppen $COOR^5$, $NHCOR^5$, $COR^5$ wobei

  $R^5$ - für Wasserstoff, Alkyl (bis $C_4$), Phenyl oder Benzyl steht,

- durch Fluor, Chlor oder $SO_3H$,
- durch die Gruppen 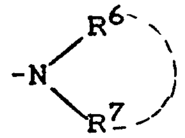, 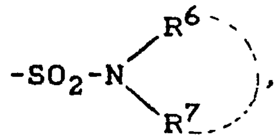,

oder 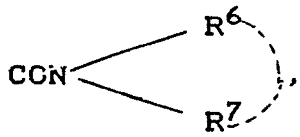,

wobei
$R^6$ und $R^7$ die oben angegebene Bedeutung haben oder
- durch die Gruppe $-SO_2-Y$ oder $-O-Y$,
wobei
Y - für Alkyl mit bis zu 4 C-Atomen, Phenyl oder Benzyl steht,

oder

b) für CHO oder $SO_3H$ steht,

c) für $COOR^5$ steht,
wobei
$R^5$ die oben angegebene Bedeutung hat, oder

d) für die Gruppe $-Z-R^8$ steht,
wobei
Z - für 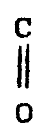 oder $-SO_2-$ steht und

$R^8$ - für geradkettiges oder verzweigtes, gesättigtes oder ungesättigtes Alkyl mit

bis zu 4 C-Atomen steht, das gegebenenfalls durch Chlor, Fluor, Hydroxy, Carboxy, $OCONH_2$, $NHCONH_2$, $SO_3H$,

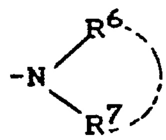 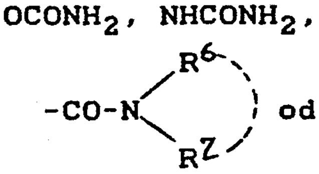 oder 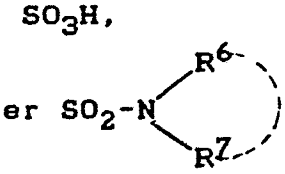

substituiert ist, wobei $R^6$ und $R^7$ die oben angegebene Bedeutung haben,

- für Phenyl,
- für Benzyl,
- für Pyridyl, Furyl, Thienyl, Thiazolyl, Isoxazolyl, Imidazolyl, Pyrazolyl steht oder
- für die Gruppe 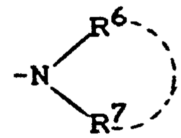 steht, wobei $R^6$, $R^7$ wiederum die oben angegebene Bedeutung haben.

Wenn die Reste 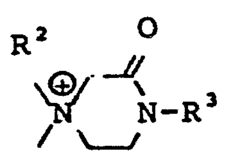 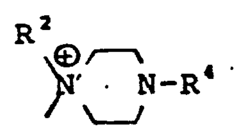

substituiert sind, dann sowohl bei den bevorzugten als auch bei den besonders bevorzugten Verbindungen der allgemeinen Formel I jeweils durch einen Substituenten wie:

a) geradkettiges, verzweigtes oder cyclisches, gesättigtes oder ungesättigtes Alkyl mit bis zu 6 C-Atomen, das gegebenenfalls wieder substituiert ist

- durch OH, CHO, $OCONH_2$, $NHCONH_2$
- durch die Gruppe $COOR^5$, $NHCOR^5$, $COR^5$

  wobei

  $R^5$ - für Wasserstoff, Alkyl mit bis zu 4 C-Atomen, Phenyl oder Benzyl steht,
- durch Fluor, Chlor, $SO_3H$
- durch die Gruppe 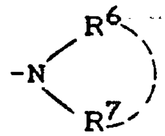 , 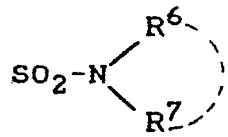

oder 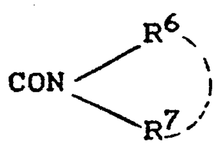,

  wobei $R^6$ und $R^7$ unabhängig voneinander für Wasserstoff, Methyl, Ethyl, Phenyl stehen oder wobei $R^6$, $R^7$ gemeinsam mit dem Stickstoffatom einen Ring bilden wie Pyrrolidin, Piperidin, Piperazin, Morpholin oder Thiomorpholin, oder
- durch die Grupope $-SO_2-Y$ oder O-Y,

  wobei

  Y - für Alkyl mit bis zu 4 C-Atomen, Phenyl oder Benzyl steht,

b) die Gruppe $-Z-R^8$

wobei

Z - für 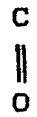 oder $-SO_2$ - steht und

$R^8$ - für geradkettiges, verzweigtes, gesättigtes oder ungesättigtes Alkyl mit bis zu 4 C-Atomen steht, das gegebenenfalls durch Fluor, Chlor, Hydroxy, Carboxy, $OCONH_2$, $NHCONH_2$, $SO_3H$,

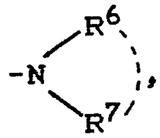, 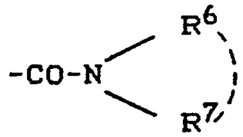 oder 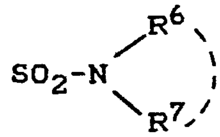

substituiert ist, wobei $R^6$, $R^7$ die oben angegebene Bedeutung haben,

- für Phenyl,
- für Benzyl,
- für Pyridyl, Thienyl, Furyl, Thiazolyl, Oxazolyl, Isoxazolyl, Imidazolyl, Pyrimidyl, Morpholinyl, Piperidinyl oder
- für die Gruppe 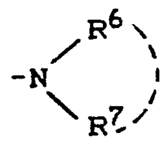 steht, wobei

$R^6$, $R^7$ wiederum die obengenannte Bedeutung haben,

c) OH, CHO, $OCONH_2$, $NHCONH_2$,

d) die Gruppen $COOR^5$, $NHCOR^5$, $COR^5$,
wobei
$R^5$ die obengenannte Bedeutung hat

e) Fluor, Chlor, $SO_3H$,

f) die Gruppe 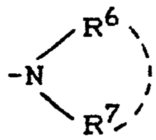

wobei $R^6$, $R^7$ wiederum die obengenannte Bedeutung haben,

oder

g) die Gruppe -O-Y
wobei
Y- für Alkyl mit bis zu 4 C-Atomen, Phenyl oder Benzyl steht.

Von den erfindungsgemäßen Verbindungen der Formel I können mehrere Isomere entstehen, welche alle antibiotisch wirksam sind und bei Bedarf durch Chromatographie oder Kristallisation getrennt werden können.

Die Verbindungen der allgemeinen Formel I können dadurch erhalten wrden, daß Verbindungen der allgemeinen Formel II

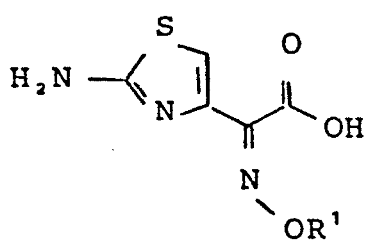

II

worin

$R^1$ die oben angegebene Bedeutung hat,

in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder Methansulfonsäurechlorid durch Überführung in das Säurehalogenid oder durch Überführung in einen aktivierten Ester mit beispielsweise N-Hydroxybenztriazol und Dicyclohexylcarbodiimid, mit Verbindungen der allgemeinen Formel III,

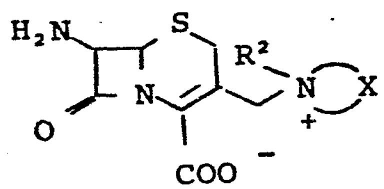

III

worin 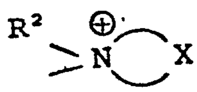 die obengenannte Bedeutung besitzt, umgesetzt werden, dann gegebenenfalls Schutzgruppen abgespalten werden und die gewünschten Salze oder aus Salzen die freien Säuren hergestellt werden.

Für die Kupplung von Carbonsäuren (II) an β-Lactame der Formel (III) kann eine große Zahl von aus der Cephalosporin- bzw. Penicillinchemie bekannten Methoden verwendet werden. Es hat sich als vorteilhaft erwiesen, die Carbonsäuren der allgemeinen Formel (II) ohne Amin-Schutzgruppe zu aktivieren und dann mit den β-Lactamen der Formel (III), die als Salze mit einem Amin in Lösung gebracht wurden, zu kuppeln.

Besonders vorteilhaft ist die Aktivierung mit Sulfonsäurederivaten der Formel V zu Anhydriden der Formel IV

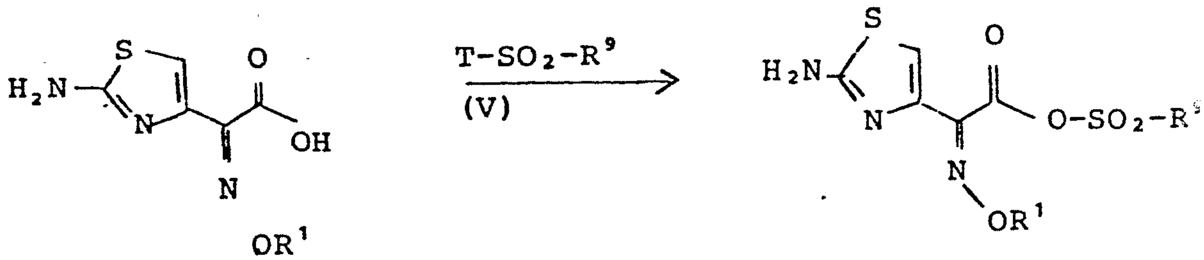


(II) (IV)

in welchen

T - für den Rest $R^9-SO_2-O-$ oder Halogen steht, und

$R^9$- für Alkyl mit bis zu 10 C-Atomen steht, das gegebenenfalls substituiert ist durch Fluor, Chlor, Cyano, Phenyl, Alkoxycarbonyl, Alkoxy oder Alkyl mit jeweils bis zu 4 C-Atomen, oder

- für Phenyl steht, das gegebenenfalls substituiert ist durch Fluor, Chlor, Brom, Cyano, Alkyl, Alkoxy, Alkylthio, Alkoxycarbonyl mit jeweils bis zu 4 C-Atomen, Nitro, Trifluormethyl oder Phenyl.

Wenn $R^9$ substituiert ist, sind bevorzugt 1 bis 3 Substituenten, besonders bevorzugt die genannten vorhanden.

Ganz besonders bevorzugt stellt $R^9$ einen Methyl- oder p-Tolylrest dar.

Die gemischten Anhydride der Formel (IV) werden hergestellt, indem man die Carbonsäuren der Formel (II) und 1-1,4-Äquivalente eines Amins in einem Lösungsmittel löst und mit 1 bis 1,2 Äquivalenten eines Sulfonsäurederivates der Formel (V) reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amine eignen sich tertiäre Amine wie z.B. Triethylamin oder Tributylamin, aber auch sterisch gehinderte sekundäre Amine wie z.B. Diisopropylamin, oder Gemische aus diesen Aminen.

Die Umsetzungen können bei Temperaturen zwischen -80°C und Raumtemperatur durchgeführt werden. Vorteilhaft wird die Aktivierung mit $Cl-SO_2CH_3$, in Dimethylformamid bei -40°C bis -60°C innerhalb von 0,2 bis 24 Stunden, vorzugsweise 0,5 bis 5 Stunden, durchgeführt.

Zum Lösen der Verbindungen der Formel (III) können die bei der Herstellung der Verbindungen der Formel (IV) genannten Lösungsmittel oder Wasser sowie als Base die dort genannten Amine verwendet werden.

Besonders vorteilhaft ist auch eine Aktivierung der Carbonsäuren der allgemeinen Formel (II) durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel (IV) eignen.

Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit einer Verbindung der Formel (III) in Form einer Lösung ihres Aminsalzes innerhalb von 2 bis 24 Stunden umgesetzt. Zum Lösen der Verbindungen der Formel (III) können die bei der Herstellung der Verbindungen der Formel (IV) genannten Lösungsmittel sowie als Base die dort genannten Amine verwendet werden.

Die Verbindungen der Formel (III) erhält man, indem man aus Verbindungen der Formel (VI) die Aminschutzgruppe $R^{10}$ abspaltet.

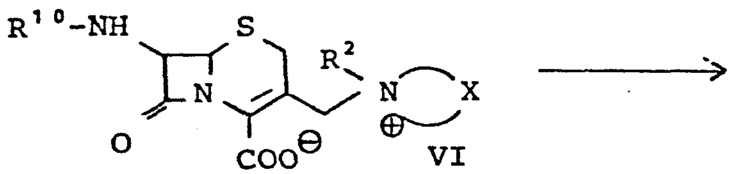

VI

(III)

Dabei kann $R^{10}$ entweder eine säurelabile Schutzgruppe wie die t-Butyloxycarbonylgruppe oder vorteilhaft eine enzymatisch abspaltbare Schutzgruppe sein. Bevorzugte enzymatisch abspaltbare Schutzgruppen sind Phenylacetyl oder 2-Thienylacetyl.

Die enzymatische Abspaltung erfolgt bei Raumtemperatur in Wasser oder einem Gemisch aus Wasser und einem polaren organischen Lösungsmittel, wie z.B. Acetonitril oder Tetrahydrofuran, mit immobilisierter Penicillin G-Acylase bei pH 7-8, bevorzugt bei pH 7,5-7,8.

Während der enzymatischen Spaltung wird der pH-Wert durch Zugabe einer Base wie Lithiumhydroxid, Natriumhydroxid, Kaliumhydroxid oder eines tert. Amins wie z.B. Triethylamin, Tripropylamin, Tributylamin oder Pyridin, konstant gehalten.

Die Verbindungen der Formel (VI) lassen sich aus Estern der Formel (VII) über Zwischenverbindungen der Formel (VIII) herstellen.

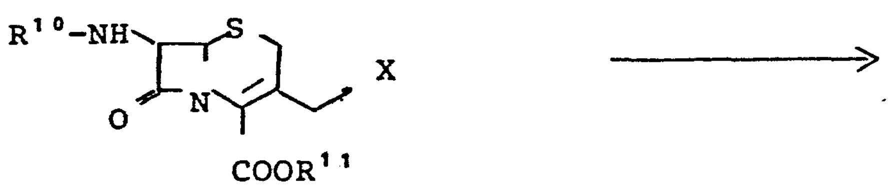

(VII)

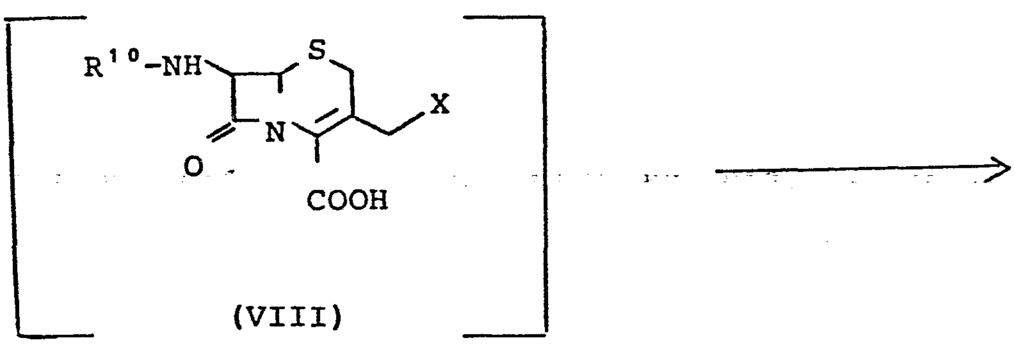

(VIII)

VI

In den Estern der Formel (VII) stellt X eine Fluchtgruppe wie Mesylat, Tosylat, Brosylat, Triflat, Nonaflat, Iodid, Bromid, Chlorid und $R^{11}$ eine in der Cephalosporinchemie übliche Säureschutzgruppe, bevorzugt eine sauer abspaltbare Schutzgruppe, wie z.B. Benzhydryl, 4-Methoxydiphenylmethyl, t-Butyl, dar,

Die Verbindungen der Formel (VII) werden durch Abspaltung der Säureschutzgruppe $R^{11}$ in die reaktiven freien Säuren der Formel (VIII) überführt. Bei den bevorzugten säurelabilen Schutzgruppen $R^{11}$ wird die Schutzgruppe in einem organischen Lösungsmittel abgespalten. Bevorzugt ist die Abspaltung der Benzhydrylschutzgruppe in Methylenchlorid mit Trifluoressigsäure möglicherweise unter Zusatz eines Alkoxybenzols, bevorzugt Methoxybenzol. Die Abspaltung erfolgt bei -20°C bis +30°C, bevorzugt bei 0°C innerhalb von 5 Minuten bis einer Stunde, bevorzugt innerhalb von 20 Minuten.

Die Säure der Formel (VIII) kann nach Abspaltung der Schutzgruppe isoliert werden. Vorteilhaft wird jedoch nicht isoliert, sondern direkt und ohne Reinigung zu Verbindungen der Formel (VI) umgesetzt. Dazu wird die bei der Reaktion (VII) ----> (VIII) entstehende Lösung von (VIII) schonend im Vakuum eingeengt. Die zurückbleibende rohe Säure wird in einem organischen Lösungsmittel, bevorzugt in Tetrahydrofuran, aufgenommen und mit 2 bis 50 Äquivalenten, bevorzugt mit 5 bis 20 Äquivalenten, eines tert. Amins der Formel

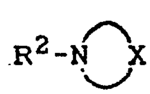 ,

wobei 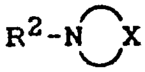 die vorstehend genannte Bedeutung hat,

zu Verbindungen der Formel (VI) umgesetzt.

Die Umsetzung wird bei Temperaturen zwischen -20°C und 40°C, bevorzugt bei 25°C, innerhalb von 10 Minuten bis 2 Stunden, bevorzugt innerhalb 30 Minuten durchgeführt. Das Produkt kann nach Beendigung der Reaktion durch Zugabe von Diethylether ausgefällt werden. Das so erhaltene Rohprodukt kann an einem Harz wie Diaion HP 20 oder XAD 7 gereinigt werden. Es ist auch vorteilhaft möglich, das Rohprodukt direkt zu Verbindungen der Formel (III) weiter umzusetzen.

Die Verbindungen der Formel (VI) lassen sich alternativ auch aus Säuren der Formel (IX) herstellen, in denen $R^{10}$ die vorstehend genannte Bedeutung hat und $R^{12}$ ein gegebenenfalls substituiertes Alkyl oder Aryl darstellt, wie Methyl, Ethyl, Propyl, Chlormethyl, Dichlormethyl, Trichlormethyl, Trifluormethyl, Phenyl. Ganz besonders bevorzugt stellt $R^{12}$ eine Methylgruppe dar.

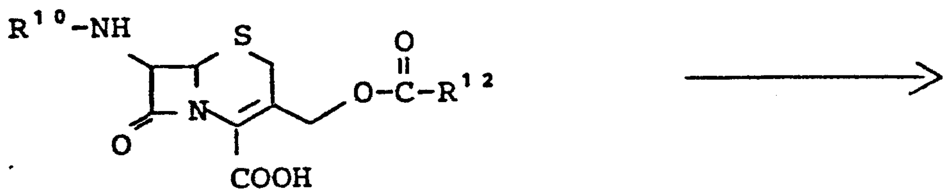

(IX)

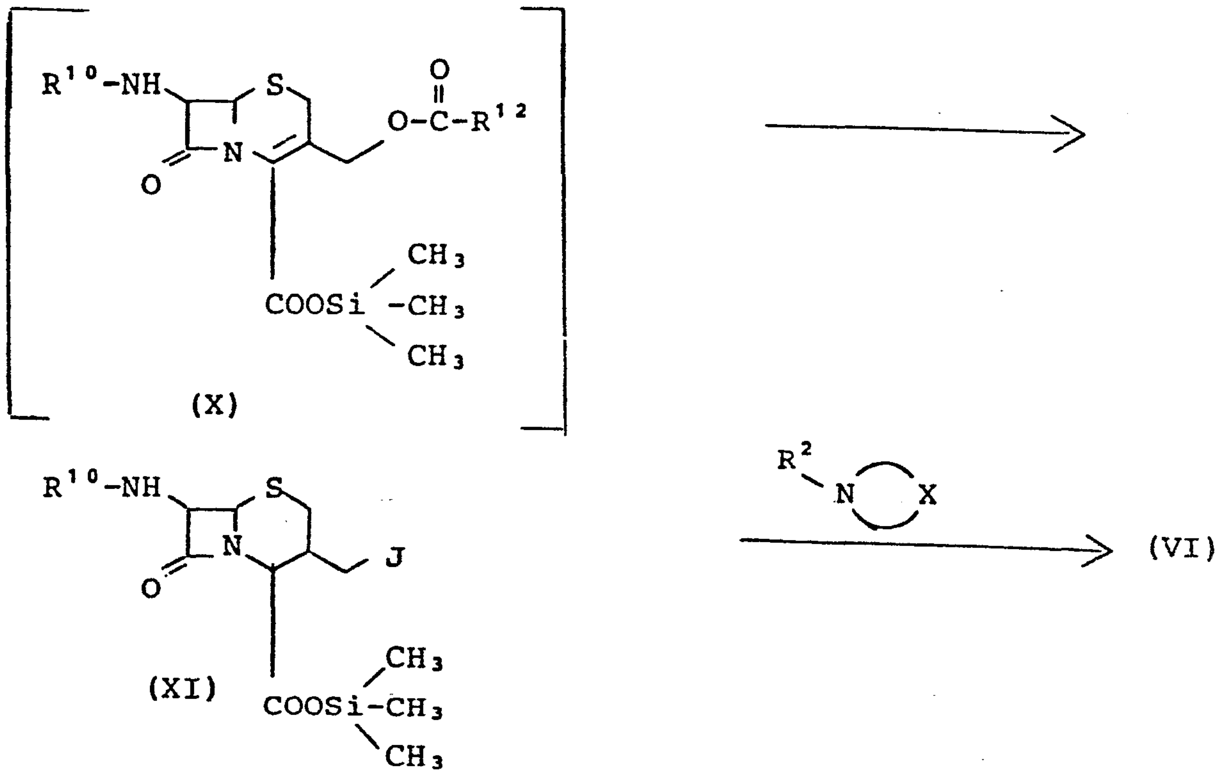

(X)

(XI)

(VI)

Die Ausgangsverbindungen der Formel (IX) werden in einem geeigneten organischen Lösungsmittel suspendiert und durch Silylierung zum Silylester X in Lösung gebracht. Besonders geeignete organische Lösungsmittel sind Chloroform, Methylenchlorid, Dichlorethan. Die Silylierung wird mit einem üblichen Silylierungsmittel wie Trimethylchlorsilan (TMCS), Hexamethyldisilazan (HMDS), N,O-Bis-(trimethylsilyl)acetamid (BSA), N,O-

Bis(trimethylsilyl)-trifluoracetamid (BSTFA), N-Methyl-N-trimethylsilylacetamid (MSA), N-Methyl-N-trimethylsilyltrifluoracetamid (MSTFA), 1,3-Bis(trimethylsilyl)harnstoff oder Trimethylsilyltrifluormethansulfonat durchgeführt.

Dabei können auch mehrere Silylierungsmittel im Gemisch eingesetzt werden.

Die Silylierung erfolgt bei -30°C bis +70°C, bevorzugt bei -10°C bis +10°C, innerhalb von 5 Minuten bis 30 Minuten. Vorteilhaft wird ein bis zu zehnfacher Überschuß des Silylierungsmittels eingesetzt, bevorzugt ein zwei- bis fünffacher Überschuß.

Die so erhaltene Lösung des Trimethylsilylesters der Formel (X) wird bei -40°C bis +30°C, bevorzugt mit drei bis vier Äquivalenten eines Trialkylsilyliodids, besonders bevorzugt Trimethylsilyliodid, innerhalb von 15 Minuten bis 2 Stunden, bevorzugt innerhalb von 30 Minuten bis 1 Stunde, zu Verbindungen der Formel (XI) umgesetzt.

Die Verbindungen der Formel XI werden vorteilhaft nicht isoliert, sondern direkt ohne Reinigung mit Aminen

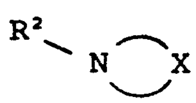 zu den Verbindungen der Formel VI

umgesetzt.

Die Verbindungen der allgemeinen Formel I können alternativ auch dadurch hergestellt werden, daß Verbindungen der Formel (XII),

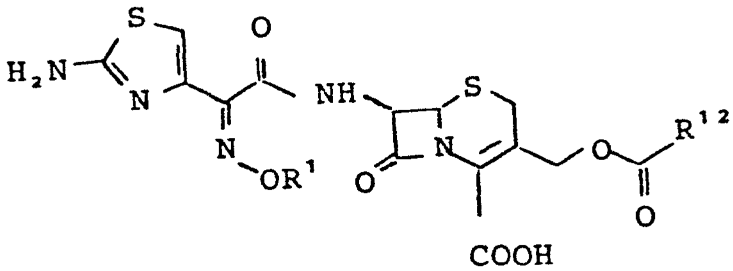

(XII)

in denen $R^1$ und $R^{12}$ die obengenannten Bedeutung haben, analog wie vorher für die Umsetzung von Verbindungen der Formel VI beschrieben, direkt ohne Isolierung der Zwischenstufen nach Silylierung und Überführung ins Iodid mit Aminen

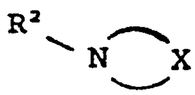

zu Verbindungen der Formel I

umgesetzt werden.

Die erfindungsgemäßen Verbindungen weisen eine starke und breite antimikrobielle Wirksamkeit besonders gegen gramnegative und grampositive Bakterien auf. Diese Eigenschaften ermöglichen ihre Verwendung als chemoterapeutische Wirkstoffe in der Medizin.

Besonders wirksam sind die erfindungsgemäßen Verbindungen gegen Bakterien und bakterienähnliche Mikroorganismen. Sie sind daher besonders gut zur Prophylaxe und

Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Graffkya tetragena (Staph. = Staphylococcus);

Lactobacteriaceae wie Streptokokken, z.B. Streptococcus pyogenes, α- bzw. β-hämolysierende Streptokokken, nicht hämolysierende Streptokokken, Str. viridans, Str. faecalis (Enterokokken) und Dipolococcus pneumoniae (Pneumokokken) (Str. = Streptococcus);

Enterobacteriaceae, wie Escherichiae-Bakterien der Coli-Gruppe: Escherichia-Bakterien, z.B. Escherichia coli, Enterobacter-Bakterien, z.B. E-Aerogenes, E-Cloacae, Klebsiella-Bakterien, z.B. K. pneumoniae, Serratia, z.B. Serratia marcescens (E. = Enterobacter) (K. = Klebsiella), Proteae-Bakterien der Proteus-Gruppe: z.B. Proteus vulgaris, Pr. morganii, Pr. rettgeri, Pr. mitabilis, (Pr. = Proteus);

Pseudomonadaceae, wie Pseudomonas-Bakterien, z.B. Pseudomonas aeruginosa, (Ps. = Pseudomonas);

Bacteroidaceae, wie Bacteroides-Bakterien, z.B. Bacteroides fragilis (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt:

Erkrankungen der Atemwege und des Rachenraumes; Otitis, Pharyngitis; Pneumonie; Peritonitis, Pyelonephritis; Cystitis; Endocarditis; Systeminfektionen; Bronchitis, Arthritis, lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen inerten pharmazeutisch geeigneten Trägerstoffen sind feste, halbfest oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten wie (a) Füll- und Streckmittel, z.B. Stärken, Milchzucker, Rohrzucker, Glukose, Mannit und Kieselsäure.

(b) Bindemittel, z.B. Carboxymethylcellulose, Alginate, Gelatine, Polyvinylpyrrolidon (c) Feuchthaltemittel, z.B. Glycerin, (d) Sprengmittel, z.G. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h)

Adsorptionsmittel, z.B. Kaolin und Bentonit und (i) Gleitmittel, z.B. Talkum, Calcium- oder Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenen Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Zur parenteralen Applikation können die Lösungen auch in steriler und blutisotonischer Form vorliegen.

Die therapeutisch wirksamen Verbindungen sollen in den oben angeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 1 bis etwa 1.000, vorzugsweise 1 bis 200 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die erfindungsgemäßen Wirkstoffe, vorzugsweise in Mengen von etwa 1 bis 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art und der Zubereitung und der Applikation des Arzneimittels sowie dem Zeitraum bzw. Intervall, innerhalb welchem die Verabreichung erfolgt. So kann es

in einigen Fällen ausreichend sein. mit weniger als der obengenannten Menge Wirkstoff auszukommen, während in anderen Fällen die oben angegebene Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen β-Lactamantibiotikum oder auch mit Aminoglykosidantibiotika, wie z.B. Gentamicin, Sisomicin, Kanamicin, Amikacin oder Tobramicin kombiniert werden.

Die erfindungsgemäßen Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht der Tiere. Besonders wertvoll erweisen sich die Wirkstoffe bei der Aufzucht und Haltung von Jung- und Masttieren. Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt:

Warmblüter wie Rinder, Schweine, Pferde, Schafe, Ziegen, Katzen, Hunde, Kaninchen; Pelztiere, z.B. Nerze und Chinchilla; Geflügel, z.B. Hühner, Gänse, Enten, Truthähne, Tauben, Papageien und Kanarienvögel, und Kaltblüter wie Fische, z.B. Karpfen, und Reptilien, z.B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen.
Die zu verabreichende Menge des Wirkstoffs sowie die entsprechende Dauer der Verabreichung hängen insbesondere von der Art, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parental erfolgen. Aus Zweckmäßigkeitsgründen ist in den meisten Fällen eine orale Verabreichung, insbesondere im Rhythmus der Nahrungs- und/oder Getränkeaufnahme der Tiere, vorzuziehen. Unter Nahrung im Sinne der vorliegenden Erfindung werden sowohl feste als auch flüssige Nahrung und auch Getränke und Wasser verstanden.

Die Wirkstoffe können als reine Stoffe oder in formulierter Form. also in Mischung mit nichttoxischen inerten Trägerstoffen beliebiger Art, z.B. mit Trägerstoffen und in Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder des Trinkwassers zugegeben wird.

Die Wirkstoffe werden nach üblichen Methoden durch einfaches Mischen als reine Stoffmischung, vorzugsweise in fein verteilter Form oder in formulierter Form in Mischung mit eßbaren nichttoxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise die Wirkstoffe in einer Gewichtkonzentration von etwa 0,01 bis 50, insbesondere 0,1 bis 10 ppm enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die Wirkstoffe neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe, gegebenenfalls auch durch ihre Oberfläche bedeckende geeignete Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält:

200 g Weizen, 340 g Mais, 361 g Sojaschrot, 60 g Rindertalg, 15 g Dicalciumphosphat, 10 g Calciumcarbonat,

4 g iodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7 H_2O$, 100 mg $FeSO_4 \times 7 H_2O$ und 20 mg $CuSO_4 \times 5 H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z.B. wie bei Kükenfutter), 30 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

0192176

## Herstellungsbeispiele

### Beispiel 1

**a) 3-[1,4-Dimethyl-3-oxo-piperazinium]methyl-7β-phenylacetamido-3-cephem-4-carboxylat**

Unter Stickstoff werden 9,36 g (24 mmol) 3-Acetoxymethyl-7β-phenylacetamido-3-cephem-4-carbonsäure bei Raumtemperatur in 100 ml absol. Methylenchlorid aufgeschlämmt und durch Zugabe von 15,2 ml (72 mmol) N-Methyl-N-trimethylsilyltrifluoracetamid (MSTFA) in Lösung gebracht. Nach Abkühlen auf 0°C werden 14 ml (96 mmol) Trimethylsilyliodid zugegeben und die Reaktionslösung wird 1 h bei 0°C gerührt. Anschließend werden 15,3 g (120 mmol) 1,4-Dimethyl-3-oxo-piperazin zugegeben und die Lösung wird 30 Minuten nachgerührt. Dann werden 4,8 ml Wasser zugegeben und nach weiteren 5 Minuten wird auf 400 ml Ether gegossen. Der Ether wird vom öligen Rückstand abdekantiert, der Rückstand nochmals mit Ether verrührt und nach erneutem Dekantieren in Wasser aufgenommen und über Absorberharz HP 20 chromatographiert (Elutionsmittel: Acetonitril/Wasser 5/95). Ausbeute 5,3 g eines Gemisches aus 2 Isomeren.

$^1$H-NMR ($D_6$-DMSO)
δ (ppm) = 9,20 [1]bd, J = 7 Hz, 7,32 [5]m, 5,61 [1]dd, J = 7 Hz, J = 5 Hz, 5,12 und 5,10 [1]d, J = 5 Hz, 5,02 [1]bd, J = 13 Hz, 4,33 und 4,18 [1]d, J = 13 Hz, 3,34-4,24 [10]m, 3,10 [3]s, 2,92 [3]s.

b) 7-Amino-3-(1,4-Dimethyl-3-oxopiperazinium)methyl-3-cephem-4-carboxylat

5,0 g 3-(1,4-Dimethyl-3-oxopiperazinium)methyl-7ß-phenylacetamido-3-cephem-4-carboxylat werden in 100 ml Wasser gelöst. Mit 4 N Triethylamin in Ethanol wird pH 7,8 eingestellt. Anschließend werden 6 g Penicillin-G-Acylase zugegeben und der pH wird durch Zugabe von Triethylamin konstant gehalten. Nach Beendigung der enzymatischen Spaltung wird von der Acylase abfiltriert und das Fitrat mit konz. Salzsäure auf pH 2 gestellt. Vom entstandenen Niederschlag wird über Kieselgelgur abgesaugt und das Filtrat wird zu 2 l Aceton getropft. Das gewünschte Produkt kristallisiert als Hydrochlorid aus und wird abgesaugt und getrocknet.
Ausbeute: 3,3 g ( x HCl x $H_2O$) als Gemisch aus zwei Isomeren.

$^1$H-NMR ($D_2O$)
δ(ppm) = 5,40 und 5,39 [1]d, J = 5 Hz, 5,19 und 5,18 [1]d, J = 5 Hz, 4,86 und 4,76 [1]d J = 13 Hz, 4,24 und 4,21 [1]d, J = 13 Hz, 4,24 [1]d, J = 18 Hz, 4,06 [1]m, 3,62 - 4,00[5]m, 3,56 und 3,54 [1]d, J = 18 Hz, 3,17 und 3,15 [3]s, 2,98 [3]s.

c) 7β-[(Z)-2-(2-Aminothiazol-4-yl)-2-methoxyiminoacetamido]-3-(1,4-dimethyl-3-oxopiperazinium)methyl-3-cephem-4-carboxylat

1,1 g (5,3 mmol) (Z)-2-(2-Aminothiazol-4-yl)-2-methoxyiminoessigsäure werden unter Stickstoff bei Raumtemperatur in 8,1 ml absol. Dimethylformamid gelöst. Nach Zugabe von 339 µl N-Ethyldiisopropylamin, 369 µl Tripropylamin und 456 µl Tributylamin wird auf -50°C gekühlt. 435 µl Methansulfonsäurechlorid werden zugegeben und die Lösung wird 30 Minuten bei -50°C gerührt. Anschließend wird diese Lösung schnell zu einer auf 0°C gekühlten Lösung von 1,6 g (4,0 mmol) 7-Amino-3-(1,4-dimethyl-3-oxopiperazinium)methyl-3-cephem-4-carboxylat (x HCl x $H_2O$) in 2,55 ml Wasser und 2,1 ml Triethylamin gegeben. Nach 5 Minuten wird die Reaktionslösung auf 500 ml Aceton gegeben. Der entstehende Niederschlag wird abgesaugt, getrocknet und über Adsorberharz HP 20 chromatographiert (Elutionsmittel: Acetonitril/Wasser 5/95). Ausbeute 700 mg eines Gemisches aus zwei Isomeren.

$^1$H-NMR ($D_6$-DMSO)
δ(ppm) = 9,63 [1]bd, J = 7 Hz, 7,26 [2]bs, 6,76 [1]s, 5,71 [1]dd, y = 7 Hz, J = 5 Hz, 5,17/5,15 [1]d, J = 5 Hz, 5,08/5,03 [1]d, J = 12 Hz, 4,32 [1]d, J = 16 Hz, 4,13 [1]d, J = 16 Hz, 4,13/4,07 [1]d, J = 12 Hz, 3,87 [3]s, 3,30 - 4,00 [6]m, 3,09 [3]bs, 2,92 [3]s.

Beispiel 2.

a) 7-Amino-3-(4-methylsulfonyl-1-methylpiperazinium)-methyl-3-cephem-4-carboxylat

Analog Beispiel 1a werden 4,68 g 3-Acetoxymethyl-7-β-phenylacetamido-3-cephem-4-carbonsäure mit 21 g 4-Methylsulfonyl-1-methylpiperazin umgesetzt. Das Produkt wird aus Aceton gefällt und direkt analog Beispiel 1b mit Penicillin G-Acylase gespalten und aufgearbeitet.
Ausbeute: 1,05 g (x HCl x $H_2O$).

$^1$H-NMR ($D_2O$)
δ(ppm) = 5,39 [1]d, J = 5 Hz, 5,17 [1]d, J = 5 Hz, 4,78 [1]d, J = 13 Hz, 4,14 [1]d, J = 13 Hz, 3,92 [1]d, J = 18 Hz, 3,40 - 3,80 [9]m, 3,10 [3]s, 3,06 [3]s.

b) 7β-[Z-2-(Aminothiazol-4-yl)-2-methoxyiminoacetamido]-3-(4-methylsulfonyl-1-methylpiperazinium)methyl-3-cephem-4-carboxylat

Analog Beispiel 1c werden 0,5 g des Produktes aus Beispiel 2a mit 0,34 g Z-2-(2-Aminothiazol-4-yl)-2-methoxyiminoessigsäure umgesetzt. Ausbeute nach Chromatographie an Adsorberharz HP 20 0,56 g.

$^{1}$H-NMR ($D_6$-DMSO)
δ(ppm) = 9,55[1]d, J = 7 Hz, 7,25 [2]bs, 6,73 [1]s, 5,66 [1]dd, J = 7 Hz, J = 5 Hz, 5,13 [1]d, J = 5 Hz, 5,14 [1]bd, J = 13 Hz, 4,06 [1]bd, J = 13 Hz, 3,83 [3]s, 3,26 [3]s, 3,20 - 4,00 [10]m, 3,04 [3]s.

Beispiel 3

a) 7-Amino-3-(4-formyl-1-methylpiperazinium)methyl-3-cephem-4-carboxylat

Analog Beipiel 1a werden 4,68 g 3-Acetoxymethyl-7-β-phenylacetamido-3-cephem-4-carbonsäure mit 15 g 4-Formyl-1-methyl-piperazin umgesetzt. Das Produkt wird aus Aceton gefällt und direkt analog Beispiel 1b mit Penicillin G-Acylase gespalten und aufgearbeitet. Ausbeute 1,2 g (x HCl x $H_2O$).

$^{1}$H-NMR ($D_2O$)
δ(ppm) = 8,02 [1]s, 5,38 [1]d, J = 5 Hz, 5,17 [1]d, J = 5 Hz, 4,78 [1]d, J = 13 Hz, 4,14 [2]d, J = 13 Hz, 3,70 - 3,98 [3]m, 3,30 - 3,64 [6]m, 3,13 [3]s.

b) 7β-[Z-2-(2-Aminothiazol-4-yl)-2-methoxyiminoacetamido]-3-(4-formyl-1-methylpiperazinium)methyl-3-cephem-4-carboxylat

Analog Beispiel 1c werden 0,6 g des Produktes aus Beispiel 3a mit 0,47 g Z-2-(2-Aminothiazol-4-yl)-2-methoxyiminoessigsäure umgesetzt. Ausbeute nach Reinigung über Adsorberharz HP 20 0,2 g.

$^1$H-NMR ($D_6$-DMSO)
δ(ppm) = 9,62 [1]d, J = 7 Hz, 8,12 [1]s, 7,26 [2]bs, 6,77 [1]s, 5,20 [1]dd, J = 7 Hz, J = 5 Hz, 5,17 [1]d, J = 5 Hz, 5,14 [1]bd, J = 13 Hz, 4,07 [1]bd, J = 13 Hz, 3,87 [3]s, 3,10 [3]s, 3,00 - 4,00 [8]m.

Beispiel 4

a) 7-Amino-3-(4-aminocarbonyl-1-methylpiperazinium)methyl-3-cephem-4-carboxylat

Analog Beispiel 1a werden 9,4 g 3-Acetoxymethyl-7-β-phenylacetamido-3-cephem-4-carbonsäure mit einer Lösung von 34 g 1-Aminocarbonyl-4-methylpiperazin in 150 ml Dimethylformamid umgesetzt. Das Produkt wird aus Ether gefällt und direkt analog Beispiel 1b mit Penicillin G-Acylase gespalten und aufgearbeitet. Ausbeute 3,1 g (x HCl x $H_2O$).

$^{1}$H-NMR ($D_2O$)

δ(ppm) = 5,39 [1]d, J = 5 Hz, 5,16 [1]d, J = 5 Hz. 4,80 [1]d, J = 13 Hz, 4,14 [1]d, J = 13 Hz, 3,85 - 4,02 [3]m, 3,35 - 3,72 [7]m, 3,22 [3]s.

b) 7β-[Z-2-(2-Aminothiazol-4-yl)-2-methoxyiminoacetamido]-3-(4-aminocarbonyl-1-methylpiperazinium)methyl-3-cephem-4-carboxylat

Analog Beispiel 1c werden 0,98 g des Produktes aus Beispiel 4a mit 0,68 g Z-2-(2-Aminothiazol-4-yl)-2-methoxyiminoessigsäure umgesetzt. Ausbeute nach Chromatographie an Adsorberharz HP 20 0,9 g.

$^{1}$H-NMR ($D_6$-DMSO)

δ(ppm) = 9,58 [1]d, J = 7 Hz, 7,53 [2]bs, 6,72 [1]s, 6,29 [2]bs, 5,66 [1]dd, J = 7 Hz, J = 5 Hz, 5,13 [1]d, J = 5 Hz, 5,09 [1]bd, J = 13 Hz, 3,99 [1]bd, J = 13 Hz, 3,82 [3]s, 3,18 - 3,92 [10]m, 3,00 [3]s.

## Beispiel 5

a) 7-Amino-3-(4-dimethylaminosulfonyl-1-methylpiperazinium)methyl-3-cephem-4-carboxylat

Analog Beispiel 1a werden 9,4 g 3-Acetoxymethyl-7-ß-phenylacetamido-3-cephem-4-carbonsäure mit 49 g 1-Dimethylaminosulfonyl-4-methyl-piperazin umgesetzt. Das Rohoprodukt wird direkt analog Beispiel 1b mit Penicillin G-Acylase gespalten.
Ausbeute 2,0 g (x HCl x $H_2O$).

$^1$H-NMR ($D_2O$)
δ(ppm) = 5,39 [1]d, J = 5 Hz, 5,96 [1]d, J = 5 Hz, 4,82 [1]d, J = 13 Hz, 4,18 [1]d, J = 13 Hz, 3,98 [1]d, J = 18 Hz, 3,48 - 3,80 [9]m, 3,13 [3]s, 2,89 [6]s.

b) 7ß-[Z-2-(2-Aminothiazol-4-yl)-2-methoxyiminoacetamido]-3-(4-dimethylaminosulfonyl-1-methylpiperazinium)methyl-3-cephem-4-carboxylat

Analog Beispiel 1c werden 0,9 g des Produktes aus Beispiel 5a mit 0,55 g Z-[2-(2-Aminothiazol-4-yl)-2-methoxyiminoessigsäure umgesetzt. Ausbeute nach Chromatographie an Adsorberharz HP 20 0,45 g.

$^1H$-NMR ($D_6$-DMSO)
δ(ppm) = 9,59 [1]d, J = 7 Hz, 7,24 [2]bs, 6,74 [1]s, 5,67 [1]dd, J = 7 Hz, J = 5 Hz, 5,13 [1]d, J = 5 Hz, 5,14 [1]bd, J = 13 Hz, 4,03 [1]bd, J = 13 Hz, 3,84 [3]s, 3,81 [1]d, J = 18 Hz, 3,20 - 3,60 [9]m, 3,04 [3]s, 2,82 [6]s.

Beispiel 6

a) 7-Amino-3-(4-acetyl-1-methylpiperazinium)methyl-3-cephem-4-carboxylat

Analog zu Beispiel 1a werden 9,4 g 3-Acetoxymethyl-7-β-phenylacetamido-3-cephem-4-carboxylat mit 34 g 1-Acetyl-4-methyl-piperazin umgesetzt. Nach Chromatographie des aus Ether gefällten Rohprodukts an Adsorberharz HP 20 erhält man 7,5 g 3-(4-Acetyl-1-methylpiperazinium)methyl-7-β-phenylacetamido-3-cephem-4-carboxylat, die anlaog zu Beispiel 1b mit Penicillin G-Acylase gespalten werden.
Ausbeute 3,2 g (x HCl x $H_2O$).

$^1H$-NMR ($D_2O$)
δ(ppm) = 5,31 [1]d, J = 5 Hz, 5,08 [1]d, J = 5 Hz, 4,80 [1]d, J = 13 Hz, 4,10 [1]d, J = 13 Hz, 3,20 - 4,00 [10]m, 3,05 [3]s, 2,03 [3]s.

b) 7β-[Z-2-(2-Aminothiazol-4-yl)-2-methoxyiminoacetamido]-3-(4-acetyl-1-methylpiperazinium)methyl-3-cephem-4-carboxylat

Analog zu Beispiel 1c werden 1,59 g des Produktes aus Beispiel 6a mit 1,24 g Z-2-(2-Aminothiazol-4-yl)-2-methoxyiminoessigsäure umgesetzt. Ausbeute nach Chromatographie an Adsorberharz HP 20 0,8 g.

$^1$H-NMR ($D_6$-DMSO)

δ(ppm) = 9,61 [1]d, J = 7 Hz, 7,26 [2]bs, 6,75 [1]s, 5,69 [1]dd, J = 7 Hz, J = 5 Hz, 5,16 [1]d, J = 5 Hz, 5,15 [1]bd, J = 13 Hz, 4,03 [1]bd, J = 13 Hz, 3,86 [3]s, 3,2 - 4,0 [10]m, 3,07 [3]s, 2,07 [3]s.

Beispiel 7

a) 7-Amino-3-(4-tert.-butoxycarbonyl-1-methylpiperazinium)methyl-3-cephem-4-carboxylat

Analog Beispiel 1a werden 9,4 g 3-Acetoxymethyl-7-β-phenylacetamido-3-cephem-4-carboxylat mit 48 g 1-Methyl-4-tert.-butoxycarbonylpiperazin umgesetzt. Das Rohprodukt wird direkt analog Beispiel 1b mit Penicillin G-Acylase gespalten. Ausbeute 1,9 g (x HCl x $H_2O$).

$^1$H-NMR ($D_2O$)

δ(ppm) = 5,29 [1]d, J = 5 Hz, 5,06 [1]d, J = 5 Hz, 4,64 [1]d, J = 13 Hz, 4,00 [1]d, J = 13 Hz, 3,75 - 4,00 [3]m, 3,20 - 3,55 [7]m, 2,98 [3]s, 1,32 [9]s.

b) 7-β-[Z-2-(2-Aminothiazol-4-yl)-2-methoxyiminoacetamido]-3-(4-tert.-butoxycarbonyl-1-methylpiperazinium)methyl-3-cephem-4-carboxylat

Analog Beispiel 1c werden 0,85 g des Produktes aus Beispiel 7a mit 0,52 g Z-2-(2-Aminothiazol-4-yl)-2-methoxyiminoessigsäure umgesetzt. Ausbeute nach Chromatographie an Adsorberharz HP 20 0,2 g.

$^1$H-NMR ($D_6$-DMSO)

δ(ppm) = 9,64 [1]d, J = 7 Hz, 7,30 [2]bs, 6,77 [1]s, 5,70 [1]dd, J = 7 Hz, J = 5 Hz, 5,17 [1]d, J = 5 Hz, 5,14 [1]bd, J = 13 Hz, 4,03 [1]bd, J = 13 Hz, 3,87 [3]s, 3,24 - 3,95 [10]m, 3,05 [3]s, 1,45 [9]s.

## Beispiel 8

a) 7-Amino-3-[4-(3-hydroxypropyl)-1-methylpiperazinium]methyl-3-cephem-4-carboxylat

Analog Beispiel 1a werden 4,68 g 3-Acetoxymethyl-7ß-phenylacetamido-3-cephem-4-carbonsäure mit 19 g 4-(3-Hydroxypropyl)-1-methyl-piperazin umgesetzt. Das Produkt wird aus Aceton gefällt und direkt analog Beispiel 1b mit Penicillin G-Acylase gespalten und aufgearbeitet.
Ausbeute: 2,3 g (x HCl x $H_2O$).

$^1$H-NMR ($D_2O$)
δ(ppm) = 5,47 [1]d, J = 5 Hz, 5,26 [1]d, J = 5 Hz, 4,97 [1]d, J = 15 Hz, 4,37 [1]d, J = 15 Hz, 4,02 [1]d, J = 18 Hz, 3,70 - 3,90 [10]m, 3,64 [1]d, J = 18 Hz, 3,45 [2]m, 3,33 [3]s, 2,04 [2]m.

b) 7ß-[Z-2-(2-Aminothiazol-4-yl)-2-methoxyiminoacetamido]-3-[4-(3-hydroxypropyl)-1-methylpiperazinium]-methyl-3-cephem-4-carboxylat

Analog Beispiel 1 c werden 1,0 g des Produktes aus Beispiel 8a mit 0,66 g Z-2-(2-Aminothiazol-4-yl)-2-methoxyiminoessigsäure umgesetzt. Ausbeute nach Chromatographie an Adsorberharz HP 20 0,68 g.

$^1$H-NMR ($D_6$-DMSO)
δ(ppm) = 9,60 [1]d, J = 7 Hz, 7,27 [2]bs, 6,74 [1]s, 5,66 [1] dd, J = 7 Hz, J = 5 Hz, 5,15 [1]d, J = 5 Hz, 5,08 [1]d, J = 14 Hz, 4,02 [1]d, J = 14 Hz, 3,83 [3]s, 3,81 [1]d, J = 18 Hz, 3,30 - 3,50 [7]m, 2,97 [3]s, 2,40 - 2,80 [6]m, 1,55 [2]m.

Beispiel 9

7β-[Z-2-(2-Aminothiazol-4-yl)-2-methoxyiminoacetamido]-3-(4-aminocarbonyl-1-ethylpiperazinium)methyl-3-cephem-4-carboxylat

$^1$H-NMR ($D_6$-DMSO)
δ (ppm)= 9,64 [1] d, J=7 Hz, 7,29 [2] bs, 6,77 [1] s, 6,33 [2] bs, 5,68 [1] dd, J=7 Hz, J= 5 Hz, 5,10 [1] d, J=13 Hz, 5,08 [1] d, J=5 Hz, 3,96 [1] d, J=13 Hz, 3,88 [3] s, 3,20 - 3,92 [12] m, 1,26 [3] m.

Beispiel 10

7β-[Z-2-(2-Aminothiazol-4-yl)-2-methoxyiminoacetamido]-3-[4-(2-hydroxyethyl)-1-methylpiperazinium]methyl-3-cephem-4-carboxylat

$^1$H-NMR ($D_6$-DMSO)
δ (ppm)= 9,64 [1] d, J=7 Hz, 7,30 [2] bs, 6,78 [1] s, 5,70 [1] dd, J=7 Hz, J= 5 Hz, 5,18 [1] d, J=5 Hz, 5,12 [1] d, J=13 Hz, 4,01 [1] d, J=13 Hz, 3,88 [3] s, 3,85 [1] d, J=18 Hz, 3,20 - 360 [7] m, 3,01 [3] s, 2,40 - 280 [6] m.

0192176

## Beispiel 11

7β-[Z-2-(2-Aminothiazol-4-yl)-2-methoxyiminoacetamido]-3-[1-methyl-4-(2-oxoimidazolidino)carbonylpiperazinium]methyl-3-cephem-4-carboxylat

$^1$H-NMR ($D_6$-DMSO)
δ (ppm)= 9,61 [1] d, J=7 Hz, 7,50 [1] bs, 7,28 [2] bs, 6,76 [1] s, 5,69 [1] dd, J=7 Hz, J= 5 Hz, 5,16 [1] d, J=13 Hz, 5,15 [1] d, J=5 Hz, 4,05 [1] d, J=13 Hz, 3,88 [3] s, 3,20 - 3,96 [14] m, 3,08 [3] s.

## Beispiel 12

7β-[Z-2-(2-Aminothiazol-4-yl)-2-methoxyiminoacetamido]-3-[1-methyl-4-(3-methylsulfonyl-2-oxoimidazolidino)carbonyl-piperazinium]methyl- 3- cephem-4-carboxylat

$^1$H-NMR ($D_6$-DMSO)
δ (ppm)= 9,58 [1] d, J=7 Hz, 7,24 [2] bs, 6,73 [1] s, 5,67 [1] dd, J=7 Hz, J=5 Hz, 5,15 [1] d, J=13 Hz, 5,12 [1] d, J=5 Hz, 4,04 [1] d, J=13 Hz, 3,84 [3] s, 3,20 - 3,98 [14] m, 3,35 [3] s, 3,08 [3] s.

## Beispiel 13

7β-[Z-2-(2-Aminothiazol-4-yl)-2-methoxyiminoacetamido]-3-[1-methyl-4-(methylphenylsulfonyl)piperazinium]methyl-3-cephem-4-carboxylat

$^1H$-NMR ($D_6$-DMSO)
δ (ppm)= 9,62 [1] d, J=7 Hz, 7,69 [2] d, J=8 Hz, 7,47 [2] d, J=8 Hz, 7,23 [2] bs, 6,72 [1] s, 5,76 [1] dd, J=7 Hz, J=5 Hz, 5,19 [1] d, J=5 Hz, 4,78 [1] d, J=13 Hz, 4,16 [1] d, J=13 Hz, 3,84 [3] s, 3,00 - 3,95 [10] m, 2,92 [3] s, 2,43 [3] s.

Patentansprüche

1. Cephalosporine der allgemeinen Formel (I)

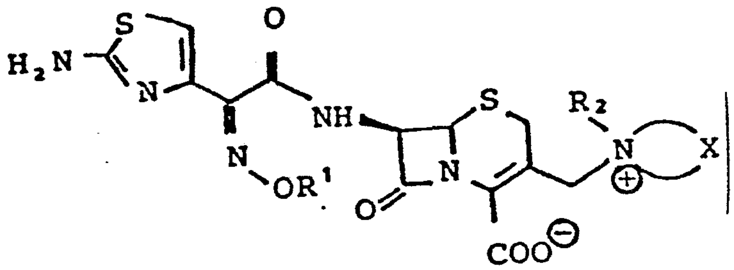 (I).

in welcher

$R^1$ - für geradkettiges oder verzweigtes. gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 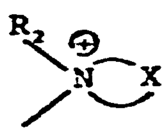 für die gegebenenfalls substituierten Gruppen 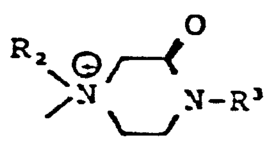

oder 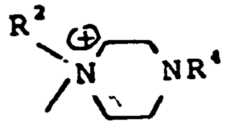 steht.

$R^3$ - für geradkettiges, verzweigtes oder cyclisches gesättigtes oder ungesättigtes Alkyl mit bis zu 10 C-Atomen steht, das gegebenenfalls sub stituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,
- durch Halogen, Cyano, $SO_3H$,

- durch die Gruppen 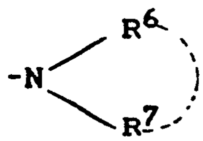 ,

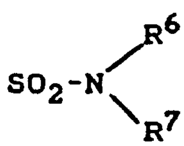 oder 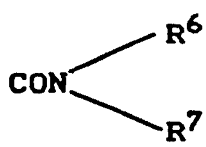 wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann
und der gegebenenfalls durch $C_1$-$C_4$-Alkyl substituiert ist,

oder

- durch die Gruppe $-SO_2-Y$ oder $-O-Y$, wobei
  Y - für $C_1$-$C_8$-Alkyl, Aryl mit 6 bis 10 C-Atomen oder
  - für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist
- durch OH, CHO, $OCONH_2$, $-NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,

- durch Halogen, Cyano, $SO_3H$,

- durch die Gruppen 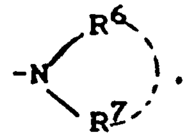

- 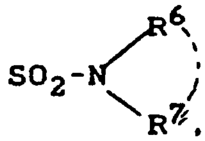 oder 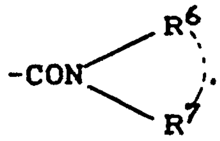

wobei $R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,

oder

- durch die Gruppe $-SO_2$-Y oder -O-Y

  wobei

  Y - für $C_1$-$C_8$-Alkyl, Aryl mit 6 bis 10 C-Atomen oder

  - für $C_7$-$C_{14}$-Aralkyl steht,

b) für CHO, $SO_3H$ steht,

c) für $COOR^5$ steht, wobei $R^5$ die oben angegebene Bedeutung hat,

oder

d) für die Gruppe $-Z-R^8$ steht,

wobei

Z - für 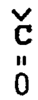 oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

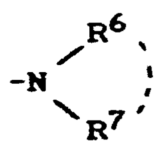 , 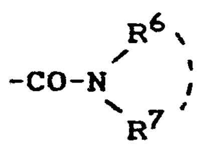 oder 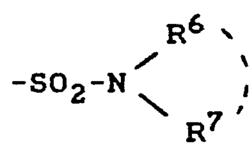

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder
- für die Gruppe 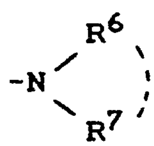 steht, wobei

$R^6$, $R^7$ die obengenannte Bedeutung haben.

2. Cephalosporine gemäß Anspruch 1, dadurch gekennzeichnet, daß die Reste

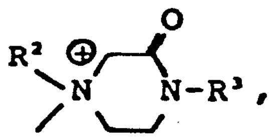, 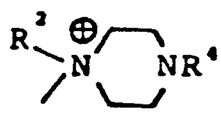

substituiert sind, durch bis zu vier gleiche oder unterschiedliche Substituenten aus der Gruppe, bestehend aus

a) geradkettigem, verzweigtem oder cyclischem gesättigtem oder ungesättigtem Alkyl mit bis zu 10 C-Atomen, das gegebenenfalls wieder substituiert ist

- durch -OH, CHO, $-OCONH_2$, $-NHCONH_2$,
- durch die Gruppen $COOR^5$, $NHCOR^5$, $COR^5$,

  wobei

  $R^5$ - für Wasserstoff, Alkyl mit bis zu 8 C-Atomen, Aryl mit 6 bis 10 C-Atomen oder $C_7$-$C_{14}$-Aralkyl steht,

- durch Halogen, Cyano, $SO_3H$,
- durch die Gruppen 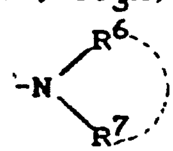 ,

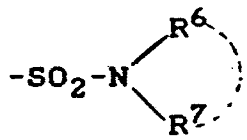 oder 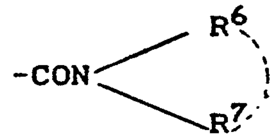

wobei

$R^6$ und $R^7$ unabhängig voneinander Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der gegebenenfalls durch $C_1$-$C_4$-Alkyl substituiert ist. oder

- durch die Gruppe $-SO_2-Y$ oder $-O-Y$
wobei
Y - für $C_1$-$C_6$-Alkyl, Aryl mit 6 bis 10 C-Atomen oder
- für $C_7$-$C_{14}$-Aralkyl steht
oder

b) der Gruppe $-Z-R^8$,
wobei

Z - für 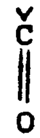 oder $-SO_2-$ steht und

$R^8$ - für geradkettiges, verzweigtes oder cyclisches gesättigtes oder ungesättigtes Alkyl mit bis zu 6 C-Atomen steht, das gegebenenfalls durch Halogen, Hydroxy, Cyano, Alkoxy, Alkylthio mit jeweils 6 C-Atomen, Aryloxy, Arylthio (6-10 C-Atome), Carboxy, $C_1$-$C_6$-Alkoxycarbonyl, $-OCONH_2-$, $-NHCONH_2$, $-SO_3H$,

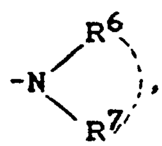, 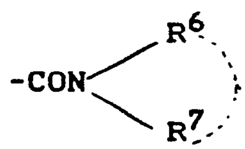 oder

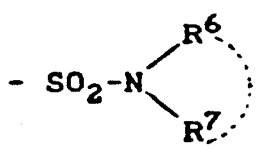

substituiert ist, wobei $R^6$, $R^7$ die oben angegebene Bedeutung haben

- für Aryl mit 6 bis 10 C-Atomen
- für $C_7$-$C_{14}$-Aralkyl
- für Heterocyclyl

oder

- für die Gruppe 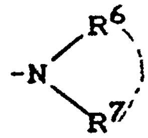 steht.

wobei $R^6$, $R^7$ wiederum die obengenannte Bedeutung haben.

c) -OH, CHO, $OCONH_2$, $NHCONH_2$,

d) den Gruppen $COOR^5$, $NHCOR^5$, $COR^5$,

wobei
$R^5$ - für Wasserstoff, Alkyl mit bis zu 8 C-Atomen, Aryl mit 6 bis 10 C-Atomen, oder $C_7$-$C_{14}$-Aralkyl steht,

e) Halogen, Cyano, $SO_3H$

f) die Gruppe 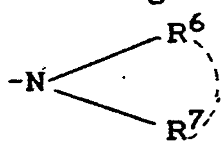 wobei

$R^6$ und $R^7$ die oben angegebene Bedeutung hat,

g) der Gruppe -O-Y, wobei

Y - für $C_1$-$C_6$-Alkyl, Aryl mit 6 bis 10 C-Atomen oder
- für $C_7$-$C_{14}$-Aralkyl steht.

3. Cephalosporine gemäß Anspruch 1, dadurch gekennzeichnet, daß

$R^1$ -für geradkettiges, verzweigtes, gesättigtes oder ungesättigtes, gegebenenfalls durch Carboxy substituiertes Alkyl mit bis zu 4 C-Atomen, steht,

$R^2$ -für geradkettiges oder verzweigtes Alkyl mit bis zu 4 C-Atomen steht, das gegebenenfalls substituiert ist durch Hydroxy, Carboxy, Methoxy, Methoxycarbonyl, Fluor, Chlor oder Brom,

der Rest 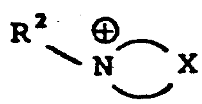 für die gegebenenfalls substituierten

Reste 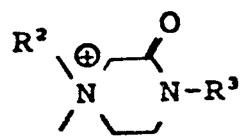 oder 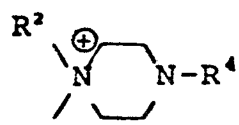 steht,

in denen

$R^3$- für geradkettiges, verzweigtes oder cyclisches, gesättigtes oder ungesättigtes Alkyl mit bis zu 8 C-Atomen steht, das gegebenenfalls substituiert ist

- durch OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 6-C-Atomen, Phenyl oder Benzyl steht,
- durch Fluor, Chlor, Brom oder $SO_3H$,

- durch die Gruppen - 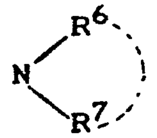 , 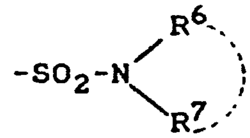

oder 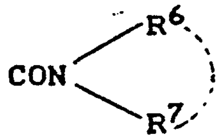 ,

wobei

$R^6$ und $R^7$ unabhängig voneinander für Wasserstoff, für $C_1$-$C_3$-Alkyl oder Phenyl stehen oder wobei die Substituenten $R^6$, $R^7$ gegebenenfalls mit dem Stickstoffatom einen 5- oder 6-gliedrigen Ring bilden, der als weitere Heteroatome ein Sauerstoff-, Schwefel- und/oder 1-2 Stickstoffatome enthalten kann und der gegebenenfalls durch $C_1$-$C_2$-Alkyl substituiert ist,

oder

- durch die Gruppe $-SO_2$-Y oder -O-Y,

wobei

Y - für $C_1$-$C_6$-Alkyl, Phenyl oder Benzyl steht,

und

$R^4$ a) für geradkettiges, verzweigtes oder cyclisches, gesättigtes oder ungesättigtes Alkyl mit bis zu 8 C-Atomen steht, das mindestens einmal substituiert ist

- durch OH, CHO, $OCONH_2$, $NHCONH_2$,
- durch die Gruppen $COOR^5$, $NHCOR^5$, $COR^5$,

wobei

$R^5$ - für Wasserstoff, Alkyl mit bis zu 6 C-Atomen, Phenyl oder Benzyl steht,

- durch Fluor, Chlor, Brom oder $SO_3H$

- durch die Gruppen 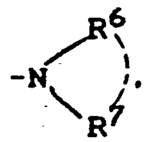, 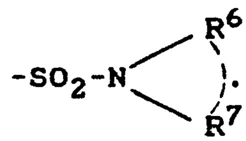,

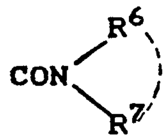

wobei $R^6$ und $R^7$ die oben angegebene Bedeutung haben

- durch die Gruppe $-SO_2-Y$ oder $-O-Y$ wobei
  Y - für $C_1$-$C_6$-Alkyl, Phenyl oder Benzyl steht, oder

b) für CHO oder $SO_3H$ steht,

c) für $COOR^5$ steht,
wobei
$R^5$ die oben angegebene Bedeutung hat,
oder

d) für die Gruppe $-Z-R^8$ steht,
wobei
Z - für 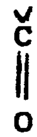 oder $-SO_2-$ steht und

$R^8$ - für geradkettiges, verzweigtes oder cyclisches, gesättigtes oder ungesättigtes Alkyl mit bis zu 6 C-Atomen, steht, das gegebenenfalls durch Fluor, Chlor, Brom, Hydroxy, Carboxy, Alkoxy oder Alkylthio mit bis zu 3 C-Atomen, Phenyloxy, Phenylthio, Alkoxycarbonyl mit bis zu 3 C-Atomen, durch

$OCONH_2$, 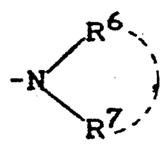 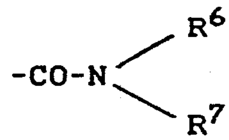

oder 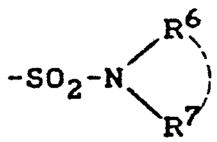, substituiert ist,

wobei $R^6$ und $R^7$ die oben angegebene Bedeutung haben,

- für Phenyl,
- für Benzyl,
- für Pyridyl, Furyl, Thienyl, Thiazolyl, Pyrimidyl, Pyrazinyl, Pyridazinyl, Isoxazolyl, Oxazolyl, Morpholinyl, Piperidinyl, Imidazolyl, Pyrazolyl, Thiazolyl oder Piperazinyl steht,

oder

- für die Gruppe 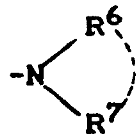 steht. wobei

$R^6$ und $R^7$ die oben angegebene Bedeutung haben.

4. Cephalosporine gemäß Anspruch 1, dadurch gekennzeichnet, daß

$R^1$ - für Methyl, Ethyl, Allyl oder 1-Carboxy-1-methylethyl steht,

$R^2$ - für Methyl, Ethyl, 2-Hydroxyethyl oder Carboxymethyl steht,

der Rest 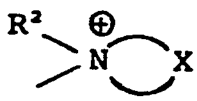 für die gegebenenfalls

substituierten Gruppen

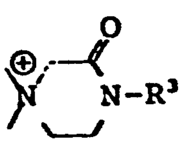 oder 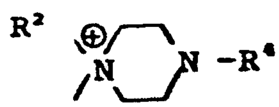 steht, worin

$R^3$ - für geradkettiges, verzweigtes oder cyclisches, gesättigtes oder ungesättigtes Alkyl mit bis zu 6 C-Atomen steht, das gegebenenfalls substituiert ist
- durch OH, CHO, $OCONH_2$, $NHCONH_2$,
- durch die Gruppen $COOR^5$, $NHCOR^5$, oder $COR^5$, wobei

$R^5$ - für Wasserstoff. Alkyl mit bis zu 4-C-Atomen, Phenyl oder Benzyl steht,

- durch Fluor. Chlor oder $SO_3H$.
- durch die Gruppen 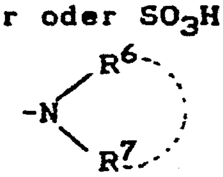 .

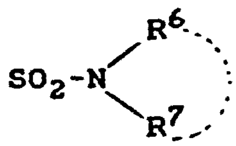 oder 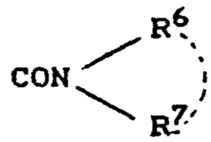 ,

wobei $R^6$, $R^7$ unabhänging voneinander für Wasserstoff, Methyl, Ethyl, Phenyl stehen oder mit dem Stickstoffatom einen Ring bilden wie Pyrrolidin, Piperazin, Piperidin, Morpholin oder Thiomorpholin, oder

- durch die Gruppe $-SO_2-Y$ oder -O-Y. wobei Y - für Alkyl mit bis zu 4 C-Atomen. Phenyl oder Benzyl steht,

und

$R^4$ a) für geradkettiges, verzweigtes oder cyclisches gesättigtes oder ungesättigtes Alkyl mit bis zu 6 C-Atomen steht, das mindestens einmal substituiert ist

- durch OH. CHO. $OCONH_2$. $NHCONH_2$
- durch die Gruppen $COOR^5$. $NHCOR^5$. $COR^5$
  wobei
  $R^5$ - für Wasserstoff. Alkyl mit bis zu 4 C-Atomen, Phenyl oder Benzyl steht,
- durch Fluor, Chlor oder $SO_3H$,
- durch die Gruppen 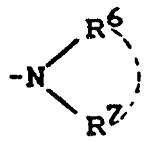, 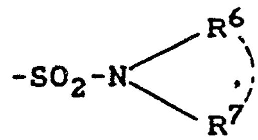

oder 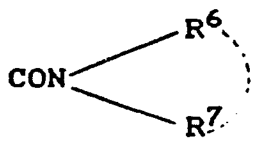 .

  wobei
  $R^6$ und $R^7$ die oben angegebene Bedeutung haben, oder
- durch die Gruppe $-SO_2-Y$ oder -O-Y,
  wobei
  Y - für Alkyl mit bis zu 4 C-Atomen, Phenyl oder Benzyl steht,

oder

b) für CHO oder $SO_3H$ steht,

c) für $COOR^5$ steht,
wobei
$R^5$ die oben angegebene Bedeutung hat, oder

d) für die Gruppe $-Z-R^8$ steht,

wobei

Z - für 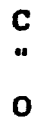 oder $-SO_2-$ steht und

$R^8$ - für geradkettiges oder verzweigtes, gesättigtes oder ungesättigtes Alkyl mit bis zu 4 C-Atomen steht, das gegebenenfalls durch Chlor, Fluor, Hydroxy, Carboxy, $OCONH_2$, $NHCONH_2$, $SO_3H$

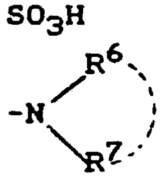 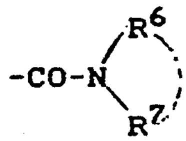 oder 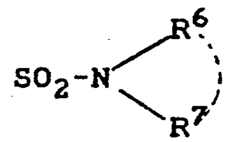

substituiert ist,

wobei $R^6$ und $R^7$ die oben angegebene Bedeutung haben,

- für Phenyl,
- für Benzyl,
- für Pyridyl, Furyl, Thienyl, Thiazolyl, Isoxazolyl, Imidazolyl, Pyrazolyl steht

oder

- für die Gruppe 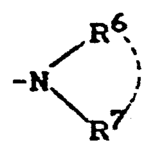 steht, wobei

$R^6$, $R^7$ wiederum die oben angegebene Bedeutung haben.

5. Cephalosporine gemäß Anspruch 1, dadurch gekennzeichnet, daß

die Reste 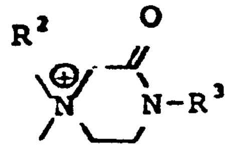 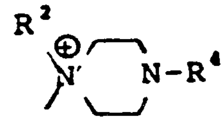

substituiert sind. durch einen Substituenten aus der Gruppe bestehend aus:

a) geradkettigem, verzweigtem oder cyclischem, gesättigtem oder ungesättigtem Alkyl mit bis zu 6 C-Atomen, das gegebenenfalls wieder substituiert ist

- durch OH, CHO, $OCONH_2$, $NHCONH_2$
- durch die Gruppe $COOR^5$, $NHCOR^5$, $COR^5$
  wobei
  $R^5$ - für Wasserstoff, Alkyl mit bis zu 4 C-Atomen, Phenyl oder Benzyl steht,
- durch Fluor, Chlor, $SO_3H$
- durch die Gruppe 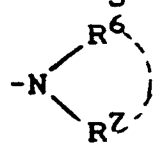 , 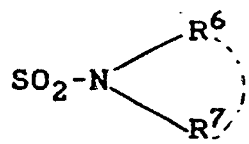

oder 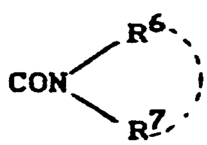 ,

wobei $R^6$ und $R^7$ unabhängig voneinander für Wasserstoff, Methyl, Ethyl, Phenyl stehen oder wobei $R^6$, $R^7$ gemeinsam mit dem Stickstoffatom einen Ring bilden wie Pyrrolidin, Piperidin, Piperazin, Morpholin oder Thiomorpholin, oder

- durch die Grupope $-SO_2-Y$ oder O-Y,
  wobei

Y - für Alkyl mit bis zu 4 C-Atomen, Phenyl oder Benzyl steht,

b) der Gruppe $-Z-R^8$
wobei
Z - für 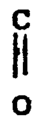 oder $-SO_2$ - steht und

$R^8$ - für geradkettiges, verzweigtes, gesättigtes oder ungesättigtes Alkyl mit bis zu 4 C-Atomen steht, das gegebenenfalls durch Fluor, Chlor, Hydroxy, Carboxy, $OCONH_2$, $NHCONH_2$, $SO_3H$,

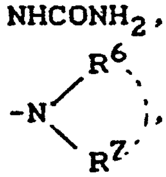, 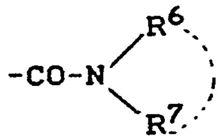 oder 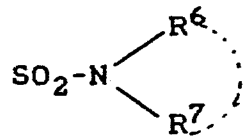

substituiert ist. wobei $R^6$, $R^7$ die oben angegebene Bedeutung haben,

- für Phenyl,
- für Benzyl,
- für Pyridyl, Thienyl, Furyl, Thiazolyl, Oxazolyl, Isoxazolyl, Imidazolyl, Pyrimidyl, Morpholinyl, Piperidinyl oder
- für die Gruppe 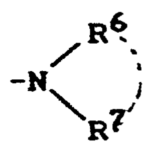 steht, wobei

$R^6$, $R^7$ wiederum die obengenannte Bedeutung haben,

c) OH, CHO, $OCONH_2$, $NHCONH_2$

d) den Gruppen $COOR^5$, $NHCOR^5$, $COR^5$, wobei $R^5$ die obengenannte Bedeutung hat

e) Fluor, Chlor, $SO_3H$,

f) der Gruppe 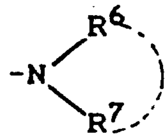

wobei $R^6$, $R^7$ wiederum die obengenannte Bedeutung haben,

oder

g) der Gruppe -O-Y wobei Y- für Alkyl mit bis zu 4 C-Atomen, Phenyl oder Benzyl steht.

6. Verfahren zur Herstellung von Cephalosporinen der allgemeinen Formel (I),

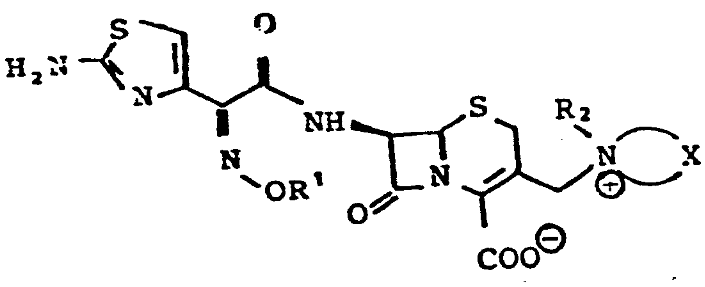

(I)

in welcher

$R^1$ - für geradkettiges oder verzweigtes. gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 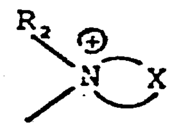 für die gegebenenfalls substituierten Gruppen 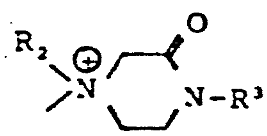

oder 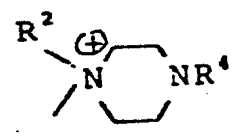

steht,

$R^3$ - für geradkettiges, verzweigtes oder cyclisches gesättigtes oder ungesättigtes Alkyl mit bis zu 10 C-Atomen steht, das gegebenenfalls substituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,

- durch Halogen, Cyano, $SO_3H$,

- durch die Gruppen 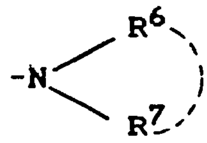 , 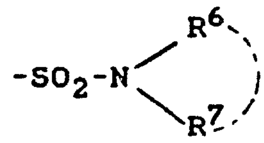

oder 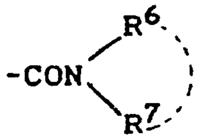 ,

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann

und der gegebenenfalls durch $C_1$-$C_4$-Alkyl substituiert ist,

- oder durch die Gruppe $-SO_2-Y$ oder -O-Y, wobei

Y - für $C_1$-$C_8$-Alkyl, Aryl mit 6 bis 10 C-Atomen oder

- für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist

- durch OH, CHO, $OCONH_2$, $-NHCONH_2$,
- durch die Gruppen $COOR^5$, $NHCOR^5$, $COR^5$, wobei

$R^5$ - für Wasserstoff, Alkyl mit bis zu 8 C-Atomen, Aryl mit 6 bis 10 C-Atomen, oder $C_7$-$C_{14}$-Aralkyl steht,

- durch Halogen, Cyano, $SO_3H$

- durch die Gruppen 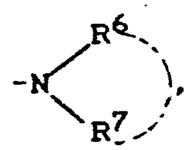,

- 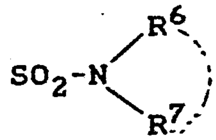 oder 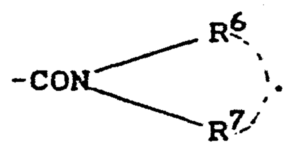.

wobei
$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,

oder

- durch die Gruppe $-SO_2$-Y oder -O-Y
wobei
Y - für $C_1$-$C_8$-Alkyl, Aryl mit 6 bis 10 men oder
- für $C_7$-$C_{14}$-Aralkyl steht,

b) für CHO, $SO_3H$ steht.

c) für $COOR^5$ steht, wobei $R^5$ die oben angegebene Bedeutung hat,

oder

d) für die Gruppe $-Z-R^8$ steht,

wobei

Z - für 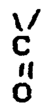 oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

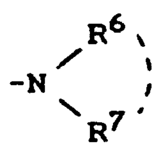 , 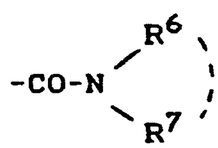 oder 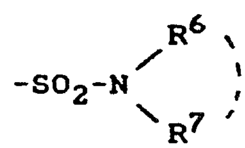

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder

- für die Gruppe 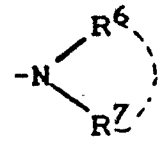 steht, wobei

$R^6$, $R^7$ die obengenannte Bedeutung haben,

dadurch gekennzeichnet, daß Verbindungen der allgemeinen Formel II

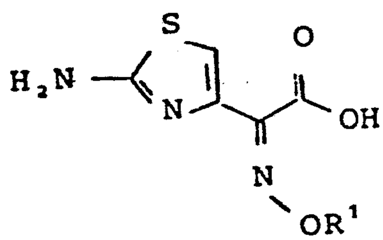

(II)

worin

$R^1$ die oben angegebene Bedeutung hat,

in denen die Aminogruppe geschützt oder ungeschützt vorliegen kann, nach Aktivierung der Carboxylgruppe durch Überführung in ein gemischtes Anhydrid, durch Überführung in das Säurehalogenid oder durch Überführung in einen aktivierten Ester, mit Verbindungen der allgemeinen Formel III,

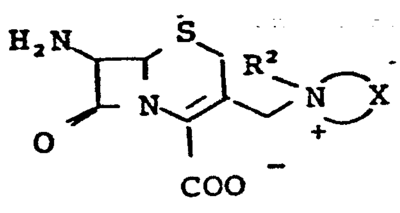

III

worin 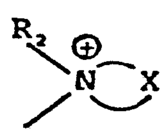 die obengenannte Bedeutung besitzt, umgesetzt werden, dann gegebenenfalls Schutzgruppen

abgespalten werden und die gewünschten Salze oder aus Salzen die freien Säuren hergestellt werden.

7. Cephalosporine der allgemeinen Formel (I)

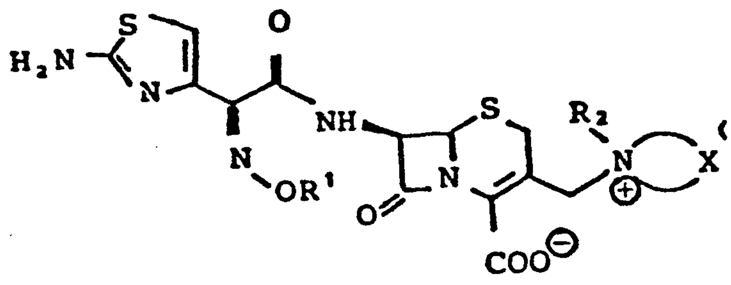 (I),

in welcher

$R^1$ - für geradkettiges oder verzweigtes, gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 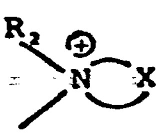 für die gegebenenfalls substituierten Gruppen 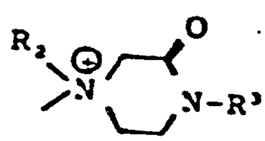

oder 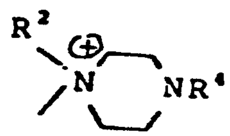 steht.

$R^3$ - für geradkettiges. verzweigtes oder cyclisches gesättigtes oder ungesättigtes Alkyl mit bis zu 10 C-Atomen steht, das gegebenenfalls substituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen oder $C_7$-$C_{14}$-Aralkyl steht,
- durch Halogen, Cyano, $SO_3H$
- durch die Gruppen 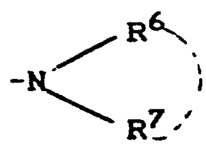 ,

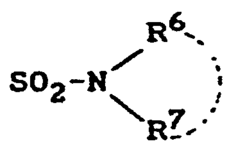 oder 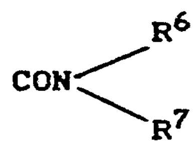

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die

Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann
und der gegebenenfalls durch $C_1$-$C_4$-Alkyl substituiert ist,

oder

- durch die Gruppe $-SO_2-Y$ oder $-O-Y$, wobei
  Y - für $C_1$-$C_8$-Alkyl, Aryl mit 6 bis 10 C-Atomen oder
  - für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist
- durch OH, CHO, $OCONH_2$, $-NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht.
- durch Halogen, Cyano, $SO_3H$.

- durch die Gruppen 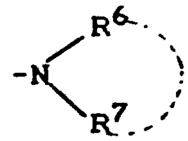 .

- 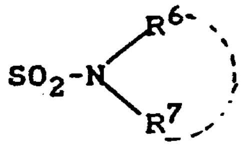 oder 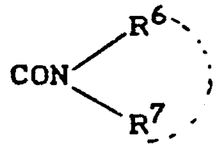

wobei
$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist.

oder

- durch die Gruppe $-SO_2-Y$ oder $-O-Y$
  wobei
  Y - für $C_1$-$C_8$-Alkyl, Aryl mit 6 bis 10 C-Atomen oder
  - für $C_7$-$C_{14}$-Aralkyl steht,

b) für CHO, $SO_3H$ steht,

c) für $COOR^5$ steht, wobei $R^5$ die oben angegebene Bedeutung hat,

oder

d) für die Gruppe $-Z-R^8$ steht,

wobei

Z - für $\overset{\vee}{\underset{\underset{O}{\|}}{C}}$ oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

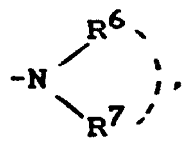, 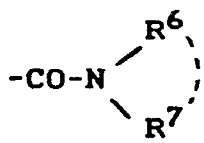 oder 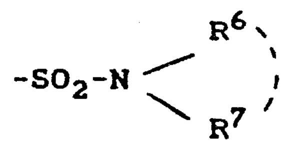

substituiert ist, wobei $R^6$, $R^7$ die oben angegebene Bedeutung haben,

- für Aryl mit 6 bis 10 C-Atomenhen
- für $C_7$-$C_{14}$-Aralkyl
- für Heterocyclyl oder
- für die Gruppe 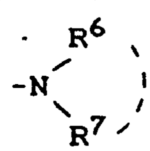 steht, wobei

$R^6$ und $R^7$ die obengenannte Bedeutung haben,

zur Anwendung bei der therapeutischen Behandlung des menschlichen und tierischen Körpers.

8. Arzneimittel enthaltend Cephalosporine der allgemeinen Formel (I)

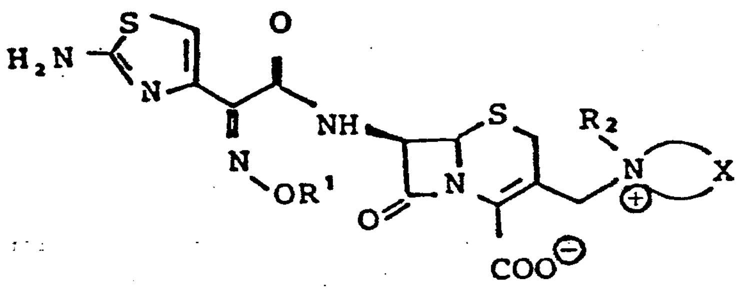 (I),

in welcher

$R^1$ - für geradkettiges oder verzweigtes, gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 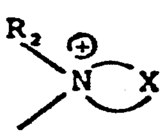 für die gegebenenfalls substituierten Gruppen 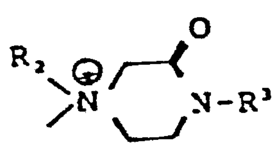

oder 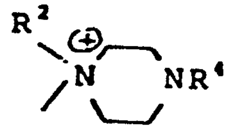 steht.

$R^3$ - für geradkettiges, verzweigtes oder cyclisches gesättigtes oder ungesättigtes Alkyl mit bis zu 10 C-Atomen steht, das gegebenenfalls substituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,
- durch Halogen, Cyano, $SO_3H$
- durch die Gruppen 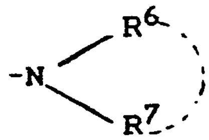 ,

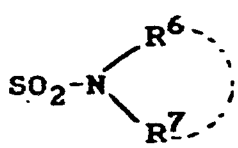 oder 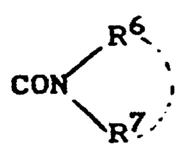

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die

0192176

Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann
und der gegebenenfalls durch $C_1$-$C_4$-Alkyl substituiert ist,

oder
- durch die Gruppe $-SO_2-Y$ oder -O-Y, wobei
  Y - für $C_1$-$C_8$-Alkyl, Aryl mit 6 bis 10 C-Atomen oder
  - für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist
- durch OH, CHO, $OCONH_2$, $-NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,

- durch Halogen, Cyano, $SO_3H$,

- durch die Gruppen 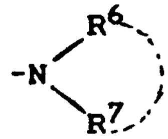 ,

- 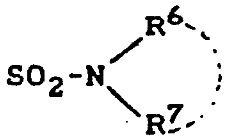 oder 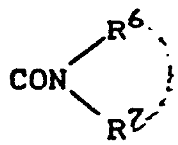

wobei
$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$. $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,

oder

- durch die Gruppe $-SO_2-Y$ oder -O-Y
  wobei
  Y - für $C_1$-$C_8$-Alkyl, Aryl mit 6 bis 10 men oder
    - für $C_7$-$C_{14}$-Aralkyl steht,

b) für CHO, $SO_3H$ steht,

c) für $COOR^5$ steht, wobei $R^5$ die oben angegebene Bedeutung hat,

oder

d) für die Gruppe $-Z-R^8$ steht,

wobei

Z - für 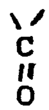 oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

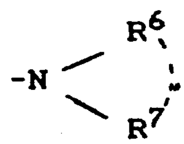 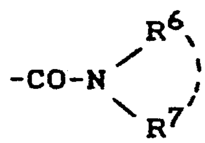 oder 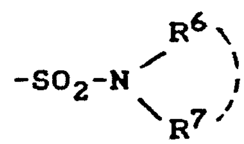

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder
- für die Gruppe 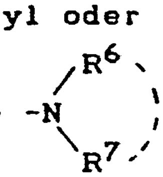 steht, wobei

- $R^6$, $R^7$ die oben genannte Bedeutung haben.

9. Verwendung von Cephalosporinen der allgemeinen Formel (I)

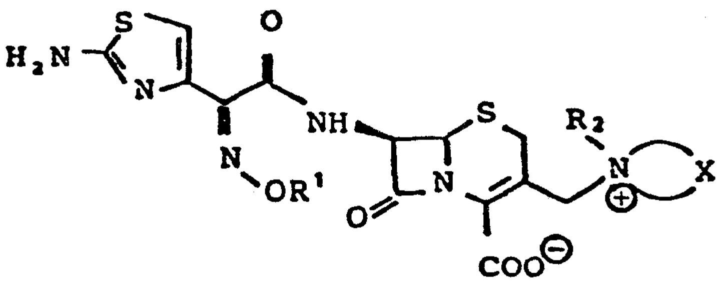 (I),

in welcher

$R^1$ - für geradkettiges oder verzweigtes, gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 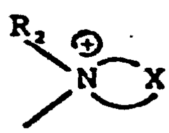 für die gegebenenfalls substituierten Gruppen 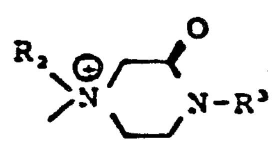

oder 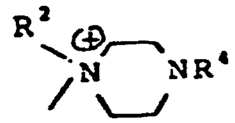

steht,

$R^3$ - für geradkettiges, verzweigtes oder cyclisches gesättigtes oder ungesättigtes Alkyl mit bis zu 10 C-Atomen steht, das gegebenenfalls substituiert ist

- durch -OH, CHO, $OCONH_2$, $NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen, oder $C_7$-$C_{14}$-Aralkyl steht,
- durch Halogen, Cyano, $SO_3H$,

- durch die Gruppen 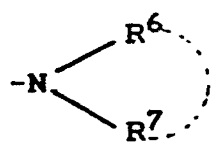 ,

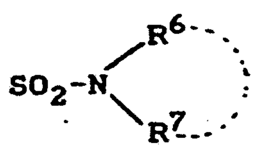 oder 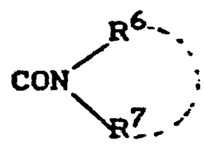

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die

Substituenten $R^6$. $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann und der gegebenenfalls durch $C_1$-$C_4$-Alkyl substituiert ist,

oder

- durch die Gruppe $-SO_2$-Y oder -O-Y, wobei
  Y - für $C_1$-$C_8$-Alkyl, Aryl mit 6 bis 10 C-Atomen
  oder
  - für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist
- durch OH, CHO, $OCONH_2$, $-NHCONH_2$,
- durch die Gruppen $COOR^5$, $NHCOR^5$, $COR^5$, wobei
  $R^5$ - für Wasserstoff, Alkyl mit bis zu 8 C-Atomen, Aryl mit 6 bis 10 C-Atomen oder $C_7$-$C_{14}$-Aralkyl steht,

- durch Halogen, Cyano, $SO_3H$,

- durch die Gruppen 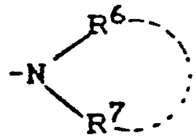 ,

- 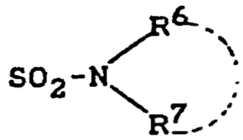 oder 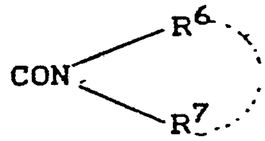

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,

oder

- durch die Gruppe $-SO_2-Y$ oder $-O-Y$
  wobei
  Y - für $C_1$-$C_8$-Alkyl, Aryl mit 6 bis 10
  men oder
  - für $C_7$-$C_{14}$-Aralkyl steht,

b) für CHO. $SO_3H$ steht.

c) für $COOR^5$ steht. wobei $R^5$ die oben angegebene Bedeutung hat,

oder

d) für die Gruppe $-Z-R^8$ steht.

wobei

Z - für 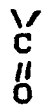 oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen. Carboxy, $C_1$-$C_6$-Alkoxycarbonyl, $-OCONH_2$, $-NHCONH_2$, $SO_3H$ oder

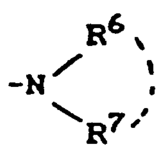 , 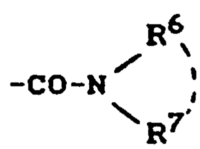 oder 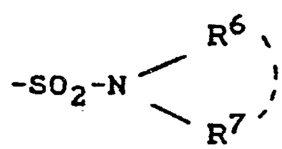

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder

- für die Gruppe 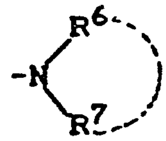 steht, wobei $R^6$ und $R^7$ die obengenannte Bedeutung haben,

bei der Herstellung von Arzneimitteln.

10. Verwendung von Cephalosporinen gemäß den Ansprüchen 1 bis 5 bei der Tierernährung.

Patentansprüche:

1. Verfahren zur Herstellung von Cephalosporinen der allgemeinen Formel (I),

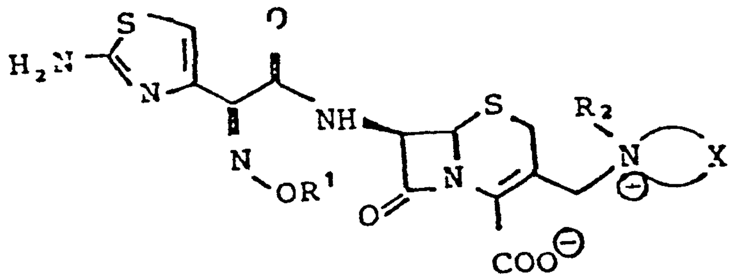 (I)

in welcher

$R^1$ - für geradkettiges oder verzweigtes, gesättigtes oder ungesättigtes, gegebenenfalls durch Carboxy substituiertes Alkyl mit bis zu 6 C-Atomen steht,

$R^2$ - für geradkettiges oder verzweigtes Alkyl mit bis zu 6 C-Atomen steht, das gegebenenfalls substituiert ist durch Hydroxy, Carboxy, Alkoxy, Alkoxycarbonyl mit jeweils bis zu 3 C-Atomen, Halogen oder Cyano,

- der Rest 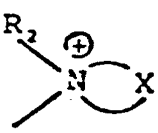 für die gegebenenfalls substituierten Gruppen 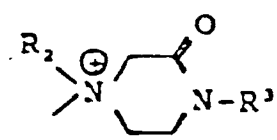

oder 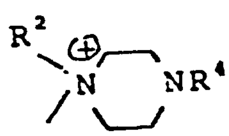

steht,

$R^3$ - für geradkettiges, verzweigtes oder cyclisches gesättigtes oder ungesättigtes Alkyl mit bis zu 10 C-Atomen steht, das gegebenenfalls substituiert ist
- durch -OH, CHO, $OCONH_2$, $NHCONH_2$,
- durch die Gruppen $COOR^5$, $NHCOR^5$, $COR^5$,

wobei

$R^5$ - für Wasserstoff, Alkyl mit bis zu 8 C-Atomen, Aryl mit 6 bis 10 C-Atomen, oder $C_7$-$C_{1\ 4}$-Aralkyl steht,

- durch Halogen, Cyano, $SO_3H$,

- durch die Gruppen 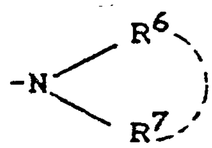 , 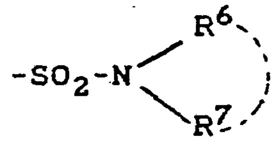

oder 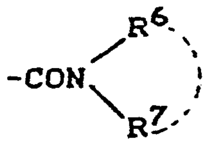 ,

wobei

$R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen, oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1-2 Stickstoffatome enthalten kann

und der gegebenenfalls durch $C_1$-$C_4$-Alkyl substituiert ist,

- oder durch die Gruppe $-SO_2-Y$ oder $-O-Y$, wobei

Y - für $C_1$-$C_8$-Alkyl, Aryl mit 6 bis 10 C-Atomen oder

- für $C_7$-$C_{14}$-Aralkyl steht,

und

$R^4$ a) für geradkettiges, verzweigtes oder cyclisches gesättigtes oder ungesättigtes Alkyl mit bis zu 10 C-Atomen steht, das mindestens einmal substituiert ist

- durch OH, CHO, $OCONH_2$, $-NHCONH_2$,
- durch die Gruppen $COOR^5$, $NHCOR^5$, $COR^5$, wobei

  $R^5$ - für Wasserstoff, Alkyl mit bis zu 8 C-Atomen, Aryl mit 6 bis 10 C-Atomen, oder $C_7$-$C_{14}$-Aralkyl steht,

- durch Halogen, Cyano, $SO_3H$

- durch die Gruppen 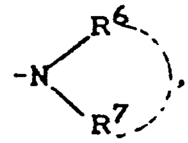,

- 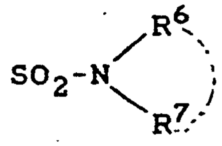 oder 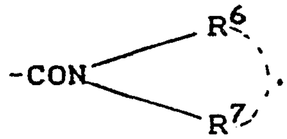.

wobei $R^6$ und $R^7$ unabhängig voneinander für Wasserstoff oder gemeinsam oder einzeln für $C_1$-$C_6$-Alkyl, $C_2$-$C_6$-Alkenyl oder Phenyl stehen oder wobei die Substituenten $R^6$, $R^7$ gegebenenfalls mit dem Stickstoffatom einen 5- bis 7-gliedrigen Ring bilden, der als weitere Heteroatome ein Sauerstoff-, Schwefel- und/oder 1 bis 2 Stickstoffatome enthalten kann und der gegebenenfalls durch $C_1$-$C_4$-Alkyl substituiert ist,

oder

- durch die Gruppe $-SO_2-Y$ oder $-O-Y$ wobei

  Y - für $C_1$-$C_8$-Alkyl, Aryl mit 6 bis 10 C-Atomen oder
  - für $C_7$-$C_{14}$-Aralkyl steht,

b) für CHO, $SO_3H$ steht.

c) für $COOR^5$ steht, wobei $R^5$ die oben angegebene Bedeutung hat,

oder

d) für die Gruppe $-Z-R^8$ steht,

wobei

Z - für 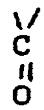 oder $-SO_2-$ steht und

$R^8$ - für geradkettiges, verzweigtes oder cyclisches gesättigtes oder ungesättigtes Alkyl mit bis zu $C_8$ steht, das gegebenenfalls durch Halogen, Hydroxy, Cyano, Alkoxy, Alkylthio mit jeweils bis zu 6 C-Atomen, Aryloxy, Arylthio mit bis zu 10 C-Atomen, Carboxy, $C_1$-$C_6$-Alkoxycarbonyl, $-OCONH_2$, $-NHCONH_2$, $SO_3H$ oder

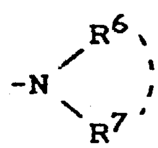 , 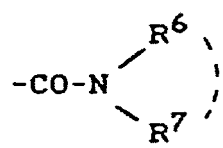 oder 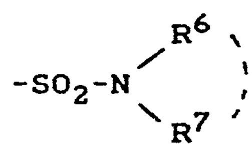

substituiert ist, wobei $R^6$, $R^7$ die oben angegebene Bedeutung haben,

- für Aryl mit 6 bis 10 C-Atomen
- für $C_7$-$C_{14}$-Aralkyl
- für Heterocyclyl oder

- für die Gruppe 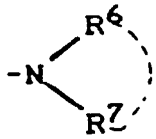 steht, wobei $R^6$, $R^7$ die obengenannte Bedeutung haben,

dadurch gekennzeichnet, daß Verbindungen der allgemeinen Formel II

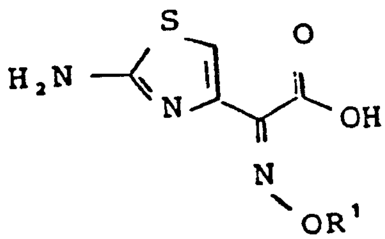

(II)

worin

$R^1$ die oben angegebene Bedeutung hat,

in denen die Aminogruppe geschützt oder ungeschützt vorliegen kann, nach Aktivierung der Carboxylgruppe durch Überführung in ein gemischtes Anhydrid, durch Überführung in das Säurehalogenid oder durch Überführung in einen aktivierten Ester, mit Verbindungen der allgemeinen Formel III,

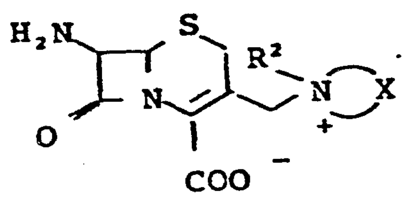

III

worin 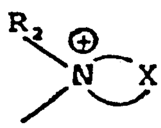 die obengenannte Bedeutung besitzt, umgesetzt werden, dann gegebenenfalls Schutzgruppen

abgespalten werden und die gewünschten Salze oder aus Salzen die freien Säuren hergestellt werden.

2. Verfahren gemäß Anspruch 6, dadurch gekennzeichnet, daß die Carbonsäuren der allgemeinen Formel (II) ohne Amin-Schutzgruppe aktiviert werden und dann mit den β-Lactamen der Formel (III), die als Salze mit einem Amin in Lösung gebracht wurden, gekuppelt werden.

3. Verfahren nach Anspruch 6, dadurch gekennzeichnet, daß die Carbonsäuren der allgemeinen Formel (II) mit Sulfonsäurederivaten der Formel (V) zu Anhydriden der Formel (IV) nach dem Schema

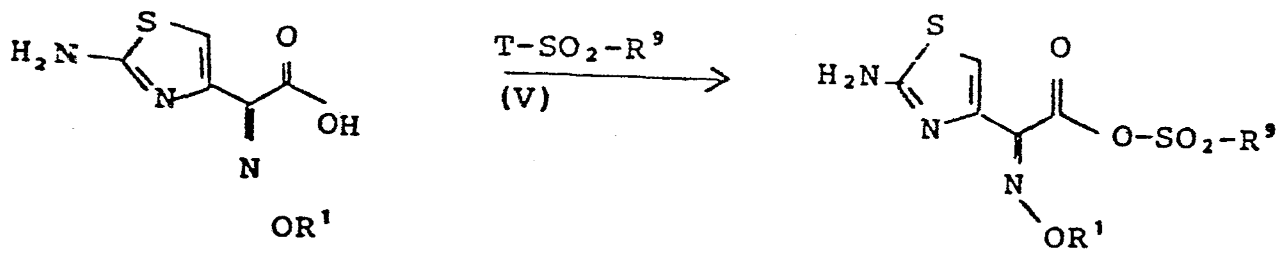


(II) (IV)

in welchen

T - für den Rest $R^9-SO_2-O-$ oder Halogen steht. und

$R^9$- für Alkyl mit bis zu 10 C-Atomen steht, das gegebenenfalls substituiert ist durch Fluor, Chlor, Cyano, Phenyl, Alkoxycarbonyl, Alkoxy oder Alkyl mit jeweils bis zu 4 C-Atomen, oder

- für Phenyl steht, das gegebenenfalls substituiert ist durch Fluor, Chlor, Brom, Cyano,

Alkyl, Alkoxy, Alkylthio, Alkoxycarbonyl mit jeweils bis zu 4 C-Atomen, Nitro, Trifluormethyl oder Phenyl, aktiviert werden.

4. Verfahren nach Anspruch 8, dadurch gekennzeichnet, daß man die Carbonsäuren der Formel (II) und 1-1,4-Äquivalente eines Amins in einem Lösungsmittel löst und mit 1 bis 1,2 Äquivalenten eines Sulfonsäurederivates der Formel (V) reagieren läßt.

5. Verfahren nach den Ansprüchen 6 bis 10, dadurch gekennzeichnet, daß als Lösungsmittel Diethylether, Tetrahydrofuran, Acetonitril, Aceton, Methylenchlorid, Chloroform oder Dimethylformamid für die Reaktion eingesetzt werden.

6. Verfahren nach den Ansprüchen 7 bis 10, dadurch gekennzeichnet, daß als Amine Triethylamin, Tributylamin oder Diisopropylamin, oder Gemische aus diesen Aminen verwendet werden.

7. Verfahren nach den Ansprüchen 6 bis 11, dadurch gekennzeichnet, daß die Umsetzungen bei Temperaturen zwischen -80°C und Raumtemperatur durchgeführt werden.

8. Verfahren nach Anspruch 7, dadurch gekennzeichnet, daß die Aktivierung der Carbonsäuren der allgemeinen Formel (II) durch Überführung in einen aktivierten Ester mit N-Hydroxysuccinimid und Dicyclohexylcarbodiimid oder mit 1-Hydroxybenztriazol und Dicyclohexylcarbodiimid erfolgt.

9. Verfahren nach Anspruch 13, dadurch gekennzeichnet, daß man die Umsetzungen bei Temperaturen zwischen -30°C und +100C durchführt.